# EUROPEAN PATENT SPECIFICATION

(11) **EP 2 121 933 B1**
(45) Date of publication and mention of the grant of the patent: **18.04.2012**
(21) Application number: 08708634.4
(22) Date of filing: 04.02.2008
(51) Int. Cl.: C12N 15/82, C12N 15/61, C12N 9/90, A01H 5/00

(54) **Use of alanine racemase genes to confer nematode resistance to plants**
Verwendung von Alanin-Racemase-Genen zur Vermittlung von Nematodenresistenz an Pflanzen
Utilisation de gènes de l'alanine racémase en vue de conférer aux plantes une résistance aux nématodes

(30) Priority: 06.02.2007 US 899746 P
(43) Date of publication of application: 25.11.2009
(73) Proprietor: BASF Plant Science GmbH, 67056 Ludwigshafen (DE)
(72) Inventor: HILL, Steve, Cary, NC 27511 (US)
(86) International application number: PCT/EP2008/051330
(87) International publication number: WO 2008/095889

(56) References cited:
- WO-A-01/94601
- WO-A-94/25606
- WO-A-03/060133
- WO-A-03/072792
- JUNG C ET AL: "New approaches to control plant parasitic nematodes" APPLIED MICROBIOLOGY AND BIOTECHNOLOGY, vol. 51, no. 4, April 1999 (1999-04), pages 439-446, XP002477823 ISSN: 0175-7598
- ONO ET AL: "Alanine racemase of alfalfa seedlings (Medicago sativa L.): First evidence for the presence of an amino acid racemase in plants" PHYTOCHEMISTRY, PERGAMON PRESS, GB, vol. 67, no. 9, May 2006 (2006-05), pages 856-860, XP005402260 ISSN: 0031-9422
- URWIN P E ET AL: "RESISTANCE TO BOTH CYST AND ROOT-KNOT NEMATODES CONFERRED BY TRANSGENIC ARABIDOPSIS EXPRESSING A MODIFIED PLANT CYSTATIN" PLANT JOURNAL, BLACKWELL SCIENTIFIC PUBLICATIONS, OXFORD, GB, vol. 12, no. 2, 1997, pages 455-461, XP000198746 ISSN: 0960-7412
- YOKOIGAWA KUMIO ET AL: "Gene cloning and characterization of alanine racemases from Shigella dysenteriae, Shigella boydii, Shigella flexneri, and Shigella sonnei" BIOCHEMICAL AND BIOPHYSICAL RESEARCH COMMUNICATIONS, vol. 288, no. 3, 2 November 2001 (2001-11-02), pages 676-684, XP002477772 ISSN: 0006-291X

### SEQUENCE LISTING

<110> BASF Plant Science GmbH
   Hill, Steve
<120> ALANINE RACEMASE LIKE NEMATODE CONTROL GENES AND METHODS OF USE IN PLANTS
<130> PF 58854
<160> 44
<170> PatentIn version 3.4
<210> 1
   <211> 33
   <212> DNA
   <213> Escherichia coli
<400> 1
   gcggcgcgcc accatgaccc gtccgataca ggc 33
<210> 2
   <211> 31
   <212> DNA
   <213> Escherichia coli
<400> 2
   gcctcgagtt acaccgtcac aaccgggacg c 31
<210> 3
   <211> 34
   <212> DNA
   <213> Escherichia coli
<400> 3
   gcggcgcgcc accatgcaag cggcaactgt tgtg 34
<210> 4
   <211> 32
   <212> DNA
   <213> Escherichia coli
<400> 4
   gcctcgagtt aatccacgta tttcatcgcg ac 32
<210> 5
   <211> 1071
   <212> DNA
   <213> Escherichia coli
<400> 5
   atgacccgtc cgatacaggc cagcctcgat ctgcaggcat taaaacagaa tctgtccatt     60
   gtccgccagg ccgcgacgca cgcgcgcgtc tggtcggtgg taaaagcgaa cgcttacggg    120
   catggtattg agcgtatctg gagcgcgatc ggggccaccg atggctttgc attgcttaac    180
   ctggaagagg caataacgtt acgtgagcgc ggctggaaag gaccgatcct gatgctggaa    240
   ggatttttcc atgctcagga tctggagatt tatgaccagc accgcctgac cacctgcgta    300
   cacagcaact ggcagctcaa agcactgcaa aatgcgcggc taaaagcacc gttggatatt    360
   tatcttaaag tgaacagtgg gatgaatcgg ttgggcttcc agcccgatcg cgtgcttacc    420
   gtctggcagc agttgcgggc aatggcgaat gttggcgaaa tgaccctgat gtcgcatttt    480
   gccgaagcgg aacatcctga tggaatttcc ggcgcgatgg cgcgtattga gcaggcggcg    540
   gaggggctgg agtgtcggcg ttcgttgtcc aattcggcgg cgactctgtg gcacccggaa    600
   gcgcattttg actgggttcg gcctggcatt attttgtatg gcgcttcgcc gtccggtcag    660
   tggcgtgata tcgccaatac cggattacgt ccggtgatga cgctaagcag tgagattatt    720
   ggtgtccaga cgctaaaagc gggcgagcgt gtgggctacg gcggtcgcta tactgcgcgc    780
   gatgaacagc gaatcggcat tgtcgccgca gggtacgccg acggttatcc gcgccacgcg    840
   cctaccggta cccctgtttt agtggacggc gtgcgcacca tgacggtggg gaccgtctcg    900
   atggatatgc tagcggtcga tttaacgcct tgcccgcagg cgggtattgg tacgccggtt    960
   gagctgtggg gcaaggagat caaaattgat gatgtcgccg ccgctgccgg aacggtgggc   1020
   tatgagttga tgtgcgcgct ggcgctacgc gtcccggttg tgacggtgta a            1071
<210> 6
   <211> 356
   <212> PRT
   <213> Escherichia coli
<400> 6
<210> 7
   <211> 1080
   <212> DNA
   <213> Escherichia coli
<400> 7
   atgcaagcgg caactgttgt gattaaccgc cgcgctctgc gacacaacct gcaacgtctt      60
   cgtgaactgg cccctgccag taaaatggtt gcggtggtga aagcgaacgc ttatggtcac     120
   ggtcttcttg agaccgcgcg aacgctcccc gatgctgacg cctttggcgt agcccgtctc     180
   gaagaagctc tgcgactgcg tgcgggggga atcaccaaac ctgtactgtt actcgaaggc     240
   ttttttgatg ccagagatct gccgacgatt tctgcgcaac attttcatac cgccgtgcat     300
   aacgaagaac agctggctgc gctggaagag gctagcctgg acgagccggt taccgtctgg     360
   atgaaactcg ataccggtat gcaccgtctg ggcgtaaggc cggaacaggc tgaggcgttt     420
   tatcatcgcc tgacccagtg caaaaacgtt cgtcagccgg tgaatatcgt cagccatttt     480
   gcgcgcgcgg atgaaccaaa atgtggcgca accgagaaac aactcgctat ctttaatacc     540
   ttttgcgaag gcaaacctgg tcaacgttcc attgccgcgt cgggtggcat tctgctgtgg     600
   ccacagtcgc attttgactg ggtgcgcccg ggcatcattc tttatggcgt ctcgccgctg     660
   gaagatcgct ccaccggtgc cgattttggc tgtcagccag tgatgtcact aacctccagc     720
   ctgattgccg tgcgtgagca taaagccgga gagcctgttg gttatggtgg aacctgggta     780
   agcgaacgtg atacccgtct tggcgtagtc gcgatgggct atggcgatgg ttatccgcgc     840
   gccgcgccgt ccggtacgcc agtgctggtg aacggtcgcg aagtaccgat tgtcgggcgc     900
   gtggcgatgg atatgatctg cgtagactta ggtccacagg cgcaggacaa agccggggat     960
   ccggtcattt tatggggcga aggtttgccc gtagaacgta tcgctgaaat gacgaaagta    1020
   agcgcttacg aacttattac gcgcctgact tcaagggtcg cgatgaaata cgtggattaa    1080
<210> 8
   <211> 359
   <212> PRT
   <213> Escherichia coli
<400> 8
   Met Gln Ala Ala Thr Val Val Ile Asn Arg Arg Ala Leu Arg His Asn
   1               5                   10                  15
   Leu Gln Arg Leu Arg Glu Leu Ala Pro Ala Ser Lys Met Val Ala Val
               20                  25                  30
   Val Lys Ala Asn Ala Tyr Gly His Gly Leu Leu Glu Thr Ala Arg Thr
           35                  40                  45
   Leu Pro Asp Ala Asp Ala Phe Gly Val Ala Arg Leu Glu Glu Ala Leu
       50                  55                  60
   Arg Leu Arg Ala Gly Gly Ile Thr Lys Pro Val Leu Leu Leu Glu Gly
   65                  70                  75                  80
   Phe Phe Asp Ala Arg Asp Leu Pro Thr Ile Ser Ala Gln His Phe His
                   85                  90                  95
   Thr Ala Val His Asn Glu Glu Gln Leu Ala Ala Leu Glu Glu Ala Ser
               100                 105                 110
   Leu Asp Glu Pro Val Thr Val Trp Met Lys Leu Asp Thr Gly Met His
           115                 120                 125
   Arg Leu Gly Val Arg Pro Glu Gln Ala Glu Ala Phe Tyr His Arg Leu
       130                 135                 140
   Thr Gln Cys Lys Asn Val Arg Gln Pro Val Asn Ile Val Ser His Phe
   145 150 155 160
   Ala Arg Ala Asp Glu Pro Lys Cys Gly Ala Thr Glu Lys Gln Leu Ala
   165 170 175
   Ile Phe Asn Thr Phe Cys Glu Gly Lys Pro Gly Gln Arg Ser Ile Ala
   180 185 190
   Ala Ser Gly Gly Ile Leu Leu Trp Pro Gln Ser His Phe Asp Trp Val
   195 200 205
   Arg Pro Gly Ile Ile Leu Tyr Gly Val Ser Pro Leu Glu Asp Arg Ser
   210 215 220
   Thr Gly Ala Asp Phe Gly Cys Gln Pro Val Met Ser Leu Thr Ser Ser
   225 230 235 240
   Leu Ile Ala Val Arg Glu His Lys Ala Gly Glu Pro Val Gly Tyr Gly
   245 250 255
   Gly Thr Trp Val Ser Glu Arg Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Pro Ile Val Gly Arg Val Ala Met Asp
   290 295 300
   Met Ile Cys Val Asp Leu Gly Pro Gln Ala Gln Asp Lys Ala Gly Asp
   305 310 315 320
   Pro Val Ile Leu Trp Gly Glu Gly Leu Pro Val Glu Arg Ile Ala Glu
   325 330 335
   Met Thr Lys Val Ser Ala Tyr Glu Leu Ile Thr Arg Leu Thr Ser Arg
   340 345 350
   Val Ala Met Lys Tyr Val Asp
   355
<210> 9
   <211> 609
   <212> DNA
   <213> Glycine max
<400> 9
   gaagccacgt catgaagagt atatcatttc agtaatgttt tgagacgcct ctataatgct 60
   ttaccaacaa aacaaaacaa aaaaaagaac atttgaaacc atttgtatta aaaaaaaaaa 120
   ggtatattag gccataatat tataggtaac atgaaatatc aaatgacacg caagagtttt 180
   gtcaaaaatg aaaccatcac acatcagaga ttatggcaaa taatgttttg tgtgtctctt 240
   gcttcaccca taacataagc ctctataact ggagagaaga aaaaaaaaag tggaggggct 300
   agggtgggaa tttggaagaa tacagttata ttgagcattg agcaagttga tagaaagctt 360
   ctcaatttgt acaaaatttg catccacatg attattaaag acgtagacag cacttcttcc 420
   ttcttttttt ctataagttt cttatatatt gttcttcatg ttttaatatt attactttat 480
   gtacgcgtct aacagtagtc ctcccaaact gctataaata gagcctcttc aacgcacctc 540
   ttggcagtac aaaaattatt catctcttct aagttctaat tttctaagca ttcagtaaaa 600
   gaactaacc 609
<210> 10
   <211> 2085
   <212> DNA
   <213> Arabidopsis thaliana
<400> 10
   cgaagagcat aagttttgtt caaatggccc aataacaaat taaaaacatg taaagtagtc     60
   agtttaaaca agcatttgca taaagtgtgg ttaatattat attaaacttc acatccaatg    120
   agcattcatg taatttaaag taactgaagt taagtatcta gaagcctttt tcttctattg    180
   gttattaatt tgcttaattt tctttataag ttaatttctg gttggtgtga aaatgtgacc    240
   ggagaaggta tctaactttt ttttttcttt aatgaattcc actaaaattt aattctgtat    300
   gtaacgcata tagtaaaatc tagaaagcga ccggcgtgcc tcctttggaa agtaatcctg    360
   taaaagtaaa agccgcgtag tgtaaaagta tatgacttct tcttcccata attattttat    420
   aattagtctt taatctaaat atttaaacat ataattcgtt ttacgagaaa gatcttcaca    480
   ctcgattagt atacattaca tttaattccc tagttcataa aatggataac aaaaggctgt    540
   gcgagattac aactgtactt gataattttg tataaatata tcctttatga atatatttta    600
   gcattgatga ccgtacatgg ttaatccagt ctgcagcata acggagtatg atattaaatg    660
   aacactttct gttcgtatca aatggtatcg aatattatta gagtgatcat tcagaagaaa    720
   aaaagagaga gaagaaaacc tacagtgtaa acattttttt ttttgctaaa tacctacagt    780
   gtaaacatga agtgctataa tttctgcaaa tagaaatcaa gaacagaaag agttgcttgg    840
   aggaaaagaa atagaaaatt aagaaatcta gtgatgtaat aaatctttcc ataaaatcaa    900
   atgtttggtc caaagtatta gttaaataat taggccacta ttcttgacaa ctctttttaa    960
   caaactcttc tatattttct cgtggtacat atgctgaaaa agatgtatgt ctaatccata   1020
   atatatctgt ataatgcgac tttcattatc tattagtacg acttctaacc tagaagataa   1080
   caagcattag ctagggcatc aaaatcaacg tggaaaaacc tacgaaaagc acgaagtgat   1140
   taatctgtgt aggggtggcg taagggtaaa gactaaagac tgagaatcta gggttcaagg   1200
   cgtaaacttg ttctgctttt tgggtttcat tttattggcg aacaacattg atgtgtgtgg   1260
   accatttggt gttcagggat tgagacaaga taatatgttt gctctcacct tctaggatta   1320
   ctcgggtgct aagactcact tagtactatt gctatatcga tatactagtt cattaccaaa   1380
   aaatggagtc ttcaaatttc gagttccaat atctgaaagc attgtttaaa gagatttgtt 1440
   ttctccctgc acaattagtt tataacttca tatatacaca atcttatcaa tttacaacca 1500
   ggtgtgtgtg aaccttcaca taatctctct tattcattca tgtatatatc caataaaagt 1560
   tcgatatgtg aaattatata tctccatcta atgttagact attcccgggt cttgactata 1620
   aatttaaagt attagacgag ctaattatat ttagcacaaa caatttcttc tgtaacagtg 1680
   tcacgcttat cactaccaaa gaataaacac tgatctgttt taatctctta ttttctcacc 1740
   catattcaaa gtcaactatt gcaagacttc gagataatta atttgatggc tatactattt 1800
   acttgacatt tgggaaaata tattttcgct gataaatttg gtttttactt ctctctccga 1860
   cggatataga aacaattcaa ttacatgcga aaatgataat tcaaccctat aaaccaaaac 1920
   aaataacaga atgcacattt ttttcaacgc gttaggtcac ctatctttca ctttagaaca 1980
   tcccttcacg tctctatata aacctcgact ctgttatcct ttgttcttca agtacaacaa 2040
   tcaactctaa gtctattata ttcaagtctt tgttttaacc taaca 2085
<210> 11
   <211> 1999
   <212> DNA
   <213> Arabidopsis thaliana
<400> 11
   gtagtgccct tcatggatac caaaagagaa aatttgattt agtgcataca tataacaata 60
   taacgccgca taataatact gtataaaaca gtcatgtaac gatatgacag cagtaataca 120
   gttccaagag acgttataat cgtatgcaat catatgcttg cgtagatttt ccaacagttt 180
   tgtttcgttg ataggaggaa ctcaacactc tagggtagtg attggtagac actattagca 240
   caaaaaatat taattttact ctgatgttta ccaaaaaagt taccaatcaa atatttaaga 300
   gatcgtactc ttccacggcg actctaaaaa ccaaagatat aggttagact cataactact 360
   ttataaagaa aatgtttaac gataactacc gagatctaat aaataaacct tcattttcaa 420
   gtatattata tttgcttctt ttgtttatat atcaaaccaa gttctggttt ataaaaatat 480
   tagataaaac tcgtctaaat aggtaggtgt aaaataaaat tttaaatttt tatcgataat 540
   atttaaaatt tgaaaagtta ataatgatcc acacattttt tctaatattt aatttagtaa 600
   tttttgtatt aaataaaatt tcaatcatat acattcgatt tttctataca ttttaactat 660
   ctatttctgc ataataaact gtattttcat tttatacgct tcatcttatg gatgatattt 720
   aaattttaaa tagtaattca tacacttttt aatatttaat ttagtatttt cttaaatcca 780
   aattttaatc ttacaattta aatatctact ttaacataat acaaatacaa tttaatttca 840
   ttgtattaaa ttcaaatata atttgattat aataaaatac aatttaattc taaaaagtcc 900
   atcttagatt ttaattttcc tttttagttt tgaaaattaa aaatttaaat ttattagata 960
   tatatgttac tttttcagtt ttcctattta tttaagaaaa aaatattttt taacacatgt 1020
   caacttgtaa acaatagact gaacacgtca ttttatatta tgtttagttt tgaaaattaa 1080
   agttaattaa atatttatat ttcttttttt tagcttttct aattattttt aaaatagtaa 1140
   atatttttaa tacaaatcaa tatctgaaca atagatttga tacataacat aatcctataa 1200
   attattaact tggaaaacga tagtttatat aataaaatta ttttcttaag ttctctaacc 1260
   ataacaatta aactatattt tagcgaagaa aagaagagaa taccgagaga acgcaacttg 1320
   cactaaaagc taccactttg gcaaatcact catttatatt attatatact atcacctcaa 1380
   ttcaatcgaa acctcaaaat aacactaata tatacacaaa gaaacaacag aataacaccg 1440
   aagaatatag gtttaggaaa atccagaatt tgttgagact aaagagatca aattttcgat 1500
   acaaggtttt gctcaatttg tattttcata ataaaattct ttatttcacc atagacttac 1560
   atgattagtt tttcttttaa taaaaaaaaa cacgcgacat gaaaattata ttatctcagt 1620
   gttgtcgaat ttgaatttga attttgagtt aaatactaca catttgttga caacttatta 1680
   aactttacaa gtctgctaca aatattgtca aatatttact aattaatgga ccaaaatcct 1740
   ctaacttgca aatttgtatc tacatcaact taaaaattag gaatatgcga cccaaaaaaa 1800
   aaaaaactag gaataataat aaaaaaatgg aatgatgtgg aggaagctct ttactctttg 1860
   agaggaagtt tataaattga ccacacattt agtctattat catcacatgt attaagactt 1920
   gacaacttgt ctttctcaca ccaaacccct ctcctctgtt tcataacatc tgctctttct   1980
   tttttttcct aagccccta                                                1999
<210> 12
   <211> 356
   <212> PRT
   <213> Escherichia coli HS
<400> 12
   Met Thr Arg Pro Ile Gln Ala Ser Leu Asp Leu Gln Ala Leu Lys Gln
   1               5                   10                  15
   Asn Leu Ser Ile Val Arg Gln Ala Ala Thr His Ala Arg Val Trp Ser
               20                  25                  30
   Val Val Lys Ala Asn Ala Tyr Gly His Gly Ile Glu Arg Ile Trp Ser
           35                  40                  45
   Ala Leu Gly Ala Thr Asp Gly Phe Ala Leu Leu Asn Leu Glu Glu Ala
       50                  55                  60
   Ile Thr Leu Arg Glu Arg Gly Trp Lys Gly Pro Ile Leu Met Leu Glu
   65                  70                  75                  80
   Gly Phe Phe His Ala Gln Asp Leu Glu Ile Tyr Asp Gln His Arg Leu
                   85                  90                  95
   Thr Thr Cys Val His Ser Asn Trp Gln Leu Lys Ala Leu Gln Asn Ala
               100                 105                 110
   Arg Leu Lys Ala Pro Leu Asp Ile Tyr Leu Lys Val Asn Ser Gly Met
           115                 120                 125
   Asn Arg Leu Gly Phe Gln Pro Asp Arg Val Leu Thr Val Trp Gln Gln
   130 135 140
   Leu Arg Ala Met Ala Asn Val Gly Glu Met Thr Leu Met Ser His Phe
   145 150 155 160
   Ala Glu Ala Glu His Pro Asp Gly Ile Ser Gly Ala Met Ala Arg Ile
   165 170 175
   Glu Gln Ala Ala Glu Gly Leu Glu Cys Arg Arg Ser Leu Ser Asn Ser
   180 185 190
   Ala Ala Thr Leu Trp His Pro Glu Ala His Phe Asp Trp Val Arg Pro
   195 200 205
   Gly Ile Ile Leu Tyr Gly Ala Ser Pro Ser Gly Gln Trp Arg Asp Ile
   210 215 220
   Ala Asn Thr Gly Leu Arg Pro Val Met Thr Leu Ser Ser Glu Ile Ile
   225 230 235 240
   Gly Val Gln Thr Leu Lys Ala Gly Glu Arg Val Gly Tyr Gly Gly Arg
   245 250 255
   Tyr Thr Ala Arg Asp Glu Gln Arg Ile Gly Ile Val Ala Ala Gly Tyr
   260 265 270
   Ala Asp Gly Tyr Pro Arg His Ala Pro Thr Gly Thr Pro Val Leu Val
   275 280 285
   Asp Gly Val Arg Thr Met Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Ala Leu Arg Val Pro
   340 345 350
   Val Val Thr Val
   355
<210> 13
   <211> 356
   <212> PRT
   <213> Escherichia coli 0157:H7 EDL933
<400> 13
   Met Thr Arg Pro Ile Gln Ala Ser Leu Asp Leu Gln Ala Leu Lys Gln
   1               5                   10                  15
   Asn Leu Ser Ile Val Arg Gln Ala Ala Pro His Ala Arg Val Trp Ser
               20                  25                  30
   Val Val Lys Ala Asn Ala Tyr Gly His Gly Ile Glu Arg Ile Trp Ser
           35                  40                  45
   Ala Leu Gly Ala Thr Asp Gly Phe Ala Leu Leu Asn Leu Glu Glu Ala
       50                  55                  60
   Ile Thr Leu Arg Glu Arg Gly Trp Lys Gly Pro Ile Leu Met Leu Glu
   65                  70                  75                  80
   Gly Phe Phe His Ala Gln Asp Leu Glu Met Tyr Asp Gln His Arg Leu
                   85                  90                  95
   Thr Thr Cys Val His Ser Asn Trp Gln Leu Lys Ala Leu Gln Asn Ala
               100                 105                 110
   Arg Leu Lys Ala Pro Leu Asp Ile Tyr Leu Lys Val Asn Ser Gly Met
           115                 120                 125
   Asn Arg Leu Gly Phe Gln Pro Asp Arg Val Leu Thr Val Trp Gln Gln
       130                 135                 140
   Leu Arg Ala Met Ala Asn Val Gly Glu Met Thr Leu Met Ser His Phe
   145                 150                 155                 160
   Ala Glu Ala Glu His Pro Asp Gly Ile Ser Gly Ala Met Ala Arg Ile
                   165                 170                 175
   Glu Gln Ala Ala Glu Gly Leu Glu Cys Arg Arg Ser Leu Ser Asn Ser
               180                 185                 190
   Ala Ala Thr Leu Trp His Pro Glu Ala His Phe Asp Trp Val Arg Pro
           195                 200                 205
   Gly Ile Ile Leu Tyr Gly Ala Ser Pro Ser Gly Gln Trp Arg Asp Ile
       210                 215                 220
   Ala Asn Thr Gly Leu Arg Pro Val Met Thr Leu Ser Ser Glu Ile Ile
   225                 230                 235                 240
   Gly Val Gln Thr Leu Lys Ala Gly Glu Arg Val Gly Tyr Gly Gly Arg
                   245                 250                 255
   Tyr Thr Ala Arg Asp Glu Gln Arg Ile Gly Ile Val Ala Ala Gly Tyr
               260                 265                 270
   Ala Asp Gly Tyr Pro Arg His Ala Pro Thr Gly Thr Pro Val Leu Val
   275 280 285
   Asp Gly Val Arg Thr Met Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Ala Leu Arg Val Pro
   340 345 350
   Val Val Thr Val
   355
<210> 14
   <211> 356
   <212> PRT
   <213> Escherichia coli UTI89
<400> 14
   Met Thr Arg Pro Ile Gln Ala Ser Leu Asp Leu Gln Ala Leu Lys Gln
   1 5 10 15
   Asn Leu Ser Ile Val Arg Gln Ala Ala Pro Arg Ala Arg Val Trp Ser
   20 25 30
   Val Val Lys Ala Asn Ala Tyr Gly His Gly Ile Glu Arg Ile Trp Ser
   35 40 45
   Ala Ile Gly Ala Thr Asp Gly Phe Ala Leu Leu Asn Leu Glu Glu Ala
   50 55 60
   Ile Thr Leu Arg Glu Arg Gly Trp Lys Gly Pro Ile Leu Met Leu Glu
   65 70 75 80
   Gly Phe Phe His Ala Gln Asp Leu Glu Ile Tyr Asp Gln His Arg Leu
   85 90 95
   Thr Thr Cys Val His Ser Asn Trp Gln Leu Lys Ala Leu Gln Asn Ala
   100 105 110
   Arg Leu Lys Ala Pro Leu Asp Ile Tyr Leu Lys Val Asn Ser Gly Met
   115 120 125
   Asn Arg Leu Gly Phe Gln Pro Asp Arg Val Leu Thr Val Trp Gln Gln
   130 135 140
   Leu Arg Ala Met Ala Asn Val Gly Glu Met Thr Leu Met Ser His Phe
   145 150 155 160
   Ala Glu Ala Glu His Pro Asp Gly Ile Ser Gly Ala Met Ala Arg Ile
                   165                 170                 175
   Glu Gln Ala Ala Glu Gly Leu Glu Cys Arg Arg Ser Leu Ser Asn Ser
               180                 185                 190
   Ala Ala Thr Leu Trp His Pro Glu Ala His Phe Asp Trp Val Arg Pro
           195                 200                 205
   Gly Ile Ile Leu Tyr Gly Ala Ser Pro Ser Gly Gln Trp Arg Asp Ile
       210                 215                 220
   Ala Asn Thr Gly Leu Arg Pro Val Met Thr Leu Ser Ser Glu Ile Ile
   225                 230                 235                 240
   Gly Val Gln Thr Leu Lys Ala Gly Glu Arg Val Gly Tyr Gly Gly Arg
                   245                 250                 255
   Tyr Thr Ala Arg Asp Glu Gln Arg Ile Gly Ile Val Ala Ala Gly Tyr
               260                 265                 270
   Ala Asp Gly Tyr Pro Arg His Ala Pro Thr Gly Thr Pro Val Leu Val
           275                 280                 285
   Asp Gly Val Arg Thr Met Thr Val Gly Thr Val Ser Met Asp Met Leu
       290                 295                 300
   Ala Val Asp Leu Thr Pro Cys Pro Gln Ala Gly Ile Gly Thr Pro Val
   305                 310                 315                 320
   Glu Leu Trp Gly Lys Glu Ile Lys Ile Asp Asp Val Ala Ala Ala Ala
                   325                 330                 335
   Gly Thr Val Gly Tyr Glu Leu Met Cys Ala Leu Ala Leu Arg Val Pro
               340                 345                 350
   Val Val Thr Val
           355
<210> 15
   <211> 356
   <212> PRT
   <213> Escherichia coli 53638
<400> 15
   Met Thr Arg Pro Ile Gln Ala Ser Leu Asp Leu Gln Ala Leu Lys Gln
   1               5                   10                  15
   Asn Leu Ser Ile Val Arg Gln Ala Ala Thr His Ala Arg Val Trp Ser
               20                  25                  30
   Val Val Lys Ala Asn Ala Tyr Gly His Gly Ile Glu Arg Ile Trp Ser
   35 40 45
   Ala Leu Gly Ala Thr Asp Gly Phe Ala Leu Leu Asn Leu Glu Glu Ala
   50 55 60
   Ile Thr Leu Arg Glu Arg Gly Trp Lys Gly Pro Ile Leu Met Leu Glu
   65 70 75 80
   Gly Phe Phe His Ala Gln Asp Leu Glu Ile Tyr Asp Gln His Arg Leu
   85 90 95
   Thr Thr Cys Val His Ser Asn Trp Gln Leu Lys Ala Leu Gln Asn Ala
   100 105 110
   Arg Leu Lys Ala Pro Leu Asp Ile Tyr Leu Lys Val Asn Ser Gly Met
   115 120 125
   Asn Arg Leu Gly Phe Gln Pro Asp Arg Val Leu Thr Val Trp Gln Gln
   130 135 140
   Leu Arg Ala Met Ala Asn Val Gly Glu Met Thr Leu Met Ser His Phe
   145 150 155 160
   Ala Glu Ala Glu His Pro Asp Gly Ile Ser Gly Ala Met Ala Arg Ile
   165 170 175
   Glu Gln Ala Ala Glu Gly Leu Glu Cys Arg Arg Ser Leu Ser Asn Ser
   180 185 190
   Ala Ala Thr Leu Trp His Pro Glu Ala His Phe Asp Trp Val Arg Pro
   195 200 205
   Gly Ile Ile Leu Tyr Gly Ala Ser Pro Ser Gly Gln Trp Arg Asp Ile
   210 215 220
   Ala Asn Thr Gly Leu Arg Pro Val Met Thr Leu Ser Ser Glu Ile Ile
   225 230 235 240
   Gly Val Gln Thr Leu Lys Ala Gly Glu Arg Val Gly Tyr Gly Gly Arg
   245 250 255
   Tyr Thr Ala Arg Asp Glu Gln Arg Ile Gly Ile Val Ala Ala Gly Tyr
   260 265 270
   Ala Asp Gly Tyr Pro Pro His Ala Pro Thr Gly Thr Pro Val Leu Val
   275 280 285
   Asp Gly Val Asn Thr Met Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Ala Leu Arg Val Pro
   340 345 350
   Val Val Thr Val
   355
<210> 16
   <211> 356
   <212> PRT
   <213> Escherichia coli E24377A
<400> 16
   Met Thr Arg Pro Ile Gln Ala Ser Leu Asp Leu Gln Ala Leu Lys Gln
   1 5 10 15
   Asn Leu Ser Ile Val Arg Gln Ala Ala Pro His Ala Arg Val Trp Ser
   20 25 30
   Val Val Lys Ala Asn Ala Tyr Gly His Gly Ile Glu Arg Ile Trp Ser
   35 40 45
   Ala Leu Gly Ala Thr Asp Gly Phe Ala Leu Leu Asn Leu Glu Glu Ala
   50 55 60
   Ile Thr Leu Arg Glu Arg Gly Trp Lys Gly Pro Ile Leu Met Leu Glu
   65 70 75 80
   Gly Phe Phe His Ala Gln Asp Leu Glu Ile Tyr Asp Gln His Arg Leu
   85 90 95
   Thr Thr Cys Val His Ser Asn Trp Gln Leu Lys Ala Leu Gln Asn Ala
   100 105 110
   Arg Leu Lys Ala Pro Leu Asp Ile Tyr Leu Lys Val Asn Ser Gly Met
   115 120 125
   Asn Arg Leu Gly Phe Gln Pro Asp Arg Val Leu Thr Val Trp Gln Gln
   130 135 140
   Leu Arg Ala Met Ala Asn Val Gly Glu Met Thr Leu Met Ser His Phe
   145 150 155 160
   Ala Glu Ala Glu His Pro Asp Gly Ile Ser Ser Ala Met Ala Arg Ile
   165 170 175
   Glu Gln Ala Ala Glu Gly Leu Glu Cys Arg Arg Ser Leu Ser Asn Ser
   180 185 190
   Ala Ala Thr Leu Trp His Pro Glu Ala His Phe Asp Trp Val Arg Pro
   195 200 205
   Gly Ile Ile Leu Tyr Gly Ala Ser Pro Ser Gly Gln Trp Arg Asp Ile
   210 215 220
   Ala Asn Thr Gly Leu Arg Pro Val Met Thr Leu Ser Ser Glu Ile Ile
   225 230 235 240
   Gly Val Gln Thr Leu Lys Ala Gly Glu Arg Val Gly Tyr Gly Gly Arg
   245 250 255
   Tyr Thr Ala Arg Asp Glu Gln Arg Ile Gly Ile Val Ala Ala Gly Tyr
   260 265 270
   Ala Asp Gly Tyr Pro Arg His Ala Pro Thr Gly Thr Pro Val Leu Val
   275 280 285
   Asp Gly Val Arg Thr Met Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Ala Leu Arg Val Pro
   340 345 350
   Val Val Thr Val
   355
<210> 17
   <211> 356
   <212> PRT
   <213> Shigella flexneri 5 str. 8401
<400> 17
   Met Thr Arg Pro Ile Gln Ala Ser Leu Asp Leu Gln Ala Leu Lys Gln
   1               5                   10                  15
   Asn Leu Ser Ile Val Arg Gln Ala Ala Pro His Ala Arg Val Trp Ser
               20                  25                  30
   Val Val Lys Ala Asn Ala Tyr Gly His Gly Ile Glu Arg Ile Trp Ser
           35                  40                  45
   Ala Leu Gly Ala Thr Asp Gly Phe Ala Leu Leu Asn Leu Glu Glu Ala
       50                  55                  60
   Ile Thr Leu Arg Glu Arg Gly Trp Lys Gly Pro Ile Leu Met Leu Glu
   65                  70                  75                  80
   Gly Phe Phe His Ala Gln Asp Leu Glu Ile Tyr Asp Gln His Arg Leu
   85 90 95
   Thr Thr Cys Val His Ser Asn Trp Gln Leu Lys Ala Leu Gln Asn Ala
   100 105 110
   Arg Leu Lys Ala Pro Leu Asp Ile Tyr Leu Lys Val Asn Ser Gly Met
   115 120 125
   Asn Arg Leu Gly Phe Gln Ser Asp Arg Val Leu Thr Val Trp Gln Gln
   130 135 140
   Leu Arg Ala Met Ala Asn Val Gly Glu Met Thr Leu Met Ser His Phe
   145 150 155 160
   Ala Glu Ala Glu His Pro Asp Gly Ile Ser Gly Ala Met Ala Arg Ile
   165 170 175
   Glu Gln Ala Ala Glu Gly Leu Glu Cys Arg Arg Ser Leu Ser Asn Ser
   180 185 190
   Ala Ala Thr Leu Trp His Pro Glu Ala His Phe Asp Trp Val Arg Pro
   195 200 205
   Gly Ile Ile Leu Tyr Gly Ala Ser Pro Ser Gly Gln Trp Arg Asp Ile
   210 215 220
   Ala Asn Thr Gly Leu Arg Pro Val Met Thr Leu Ser Ser Glu Ile Ile
   225 230 235 240
   Gly Val Gln Thr Leu Lys Ala Gly Glu Arg Val Gly Tyr Gly Gly Arg
   245 250 255
   Tyr Thr Ala Arg Asp Glu Gln Arg Ile Gly Ile Val Ala Ala Gly Tyr
   260 265 270
   Ala Asp Gly Tyr Pro Arg His Ala Pro Thr Gly Thr Pro Val Leu Val
   275 280 285
   Asp Gly Val Arg Thr Met Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Ala Leu Arg Val Pro
   340 345 350
   Val Val Thr Val
   355
<210> 18
   <211> 356
   <212> PRT
   <213> Shigella sonnei Ss046
<400> 18
   Met Thr Arg Pro Ile Gln Ala Ser Leu Asp Leu Gln Ala Leu Lys Gln
   1 5 10 15
   Asn Leu Ser Ile Val Arg Gln Ala Ala Pro His Ala Arg Val Trp Ser
   20 25 30
   Val Val Lys Ala Asn Ala Tyr Gly His Gly Ile Glu Arg Ile Trp Ser
   35 40 45
   Ala Leu Gly Ala Thr Asp Gly Phe Ala Leu Leu Asn Leu Glu Glu Ala
   50 55 60
   Ile Thr Leu Arg Glu Arg Gly Trp Lys Gly Pro Ile Leu Met Leu Glu
   65 70 75 80
   Gly Phe Phe His Ala Gln Asp Leu Glu Ile Tyr Asp Gln His Arg Leu
   85 90 95
   Thr Thr Cys Val His Ser Asn Trp Gln Leu Lys Ala Leu Gln Asn Ala
   100 105 110
   Arg Leu Lys Ala Pro Leu Asp Ile Tyr Leu Lys Val Asn Ser Gly Met
   115 120 125
   Asn Arg Leu Gly Phe Gln Pro Asp Arg Val Leu Thr Val Trp Gln Gln
   130 135 140
   Leu Arg Ala Met Ala Asn Val Gly Glu Met Thr Leu Met Ser His Phe
   145 150 155 160
   Ala Glu Ala Glu His Pro Asp Gly Ile Ser Ser Ala Met Ala Arg Ile
   165 170 175
   Glu Gln Ala Ala Glu Gly Leu Glu Cys Arg Arg Ser Leu Ser Asn Ser
   180 185 190
   Ala Ala Thr Leu Trp His Pro Glu Ala His Phe Asp Trp Val Arg Pro
   195 200 205
   Gly Ile Ile Leu Tyr Gly Ala Ser Pro Ser Gly Gln Trp Arg Asp Ile
   210 215 220
   Ala Asn Thr Gly Leu Arg Pro Val Met Thr Leu Ser Ser Glu Ile Ile
   225 230 235 240
   Gly Val Gln Thr Leu Lys Ala Gly Glu Arg Val Gly Tyr Gly Gly Arg
   245 250 255
   Tyr Thr Ala Arg Asp Glu Gln Arg Ile Gly Ile Val Ala Ala Gly Tyr
   260 265 270
   Ala Asp Gly Tyr Pro Arg His Ala Pro Thr Gly Thr Pro Val Leu Val
   275 280 285
   Asp Gly Val Arg Thr Met Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Val Leu Arg Val Pro
   340 345 350
   Val Val Thr Val
   355
<210> 19
   <211> 356
   <212> PRT
   <213> Escherichia coli B7A
<400> 19
   Met Thr Arg Pro Ile Gln Ala Ser Leu Asp Leu Gln Ala Leu Lys Gln
   1 5 10 15
   Asn Leu Ser Ile Val Arg Gln Ala Ala Pro His Ala Arg Val Trp Ser
   20 25 30
   Val Val Lys Ala Asn Ala Tyr Gly His Gly Ile Glu Arg Ile Trp Ser
   35 40 45
   Ala Leu Gly Ala Thr Asp Gly Phe Ala Leu Leu Asn Leu Glu Glu Ala
   50 55 60
   Ile Thr Leu Arg Glu Arg Gly Trp Lys Gly Pro Ile Leu Met Leu Glu
   65 70 75 80
   Gly Phe Phe His Ala Gln Asp Leu Glu Ile Tyr Asp Gln His Arg Leu
   85 90 95
   Thr Thr Cys Val His Ser Asn Trp Gln Leu Lys Ala Leu Gln Asn Ala
   100 105 110
   Arg Leu Lys Ala Pro Leu Asp Ile Tyr Leu Lys Val Asn Ser Gly Met
   115 120 125
   Asn Arg Leu Gly Phe Gln Pro Asp Arg Val Leu Thr Val Trp Gln Gln
   130 135 140
   Leu Arg Ala Met Ala Asn Val Gly Glu Met Thr Leu Met Ser His Phe
   145 150 155 160
   Ala Glu Ala Glu His Pro Asp Gly Ile Ser Ser Ala Met Ala Arg Ile
   165 170 175
   Glu Gln Ala Ala Glu Gly Leu Glu Cys Arg Arg Ser Leu Ser Asn Ser
   180 185 190
   Ala Ala Thr Leu Trp His Pro Glu Ala His Phe Asp Trp Val Arg Pro
   195 200 205
   Gly Ile Ile Leu Tyr Gly Ala Ser Pro Ser Gly Gln Trp Arg Asp Ile
   210 215 220
   Ala Asn Thr Gly Leu Arg Pro Val Met Thr Leu Ser Ser Glu Ile Ile
   225 230 235 240
   Gly Val Gln Thr Leu Lys Ala Gly Glu Arg Val Gly Tyr Gly Gly Arg
   245 250 255
   Tyr Thr Ala Arg Asp Glu Gln Arg Ile Gly Ile Val Ala Ala Gly Tyr
   260 265 270
   Ala Asp Gly Tyr Pro Arg His Ala Pro Thr Gly Thr Pro Val Leu Val
   275 280 285
   Asp Gly Val Arg Thr Met Thr Val Gly Ala Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Ala Leu Arg Val Pro
   340 345 350
   Val Val Thr Val
   355
<210> 20
   <211> 356
   <212> PRT
   <213> Escherichia coli
<400> 20
   Met Thr Arg Pro Ile Gln Ala Ser Leu Asp Leu Gln Ala Leu Lys Gln
   1               5                   10                  15
   Asn Leu Ser Ile Val Arg Gln Ala Ala Thr His Ala Arg Val Trp Ser
               20                  25                  30
   Val Val Lys Ala Asn Ala Tyr Gly His Gly Ile Glu Arg Ile Trp Ser
           35                  40                  45
   Ala Ile Gly Ala Thr Asp Gly Phe Ala Leu Leu Asn Leu Glu Glu Ala
       50                  55                  60
   Ile Thr Leu Arg Glu Arg Gly Trp Lys Gly Pro Ile Leu Met Leu Glu
   65                  70                  75                  80
   Gly Phe Phe His Ala Gln Asp Leu Glu Ile Tyr Asp Gln His Arg Leu
                   85                  90                  95
   Thr Thr Cys Val His Ser Asn Trp Gln Leu Lys Ala Leu Gln Asn Ala
               100                 105                 110
   Arg Leu Lys Ala Pro Leu Asp Ile Tyr Leu Lys Val Asn Ser Gly Met
           115                 120                 125
   Asn Arg Leu Gly Phe Gln Pro Asp Arg Val Leu Thr Val Trp Gln Gln
       130                 135                 140
   Leu Arg Ala Met Ala Asn Val Gly Glu Met Thr Leu Met Ser His Phe
   145                 150                 155                 160
   Ala Glu Ala Glu His Pro Asp Gly Ile Ser Gly Arg Met Ala Arg Ile
                   165                 170                 175
   Glu Gln Ala Ala Glu Gly Leu Glu Cys Arg Arg Ser Leu Ser Asn Ser
               180                 185                 190
   Ala Ala Thr Leu Trp His Pro Glu Ala His Phe Asp Trp Val Arg Pro
           195                 200                 205
   Gly Ile Ile Leu Tyr Gly Arg Ser Pro Ser Gly Gln Trp Arg Asp Ile
       210                 215                 220
   Ala Asn Thr Gly Leu Arg Pro Val Met Thr Leu Ser Ser Glu Ile Ile
   225                 230                 235                 240
   Gly Val Gln Thr Leu Lys Ala Gly Glu Arg Val Gly Tyr Gly Gly Arg
                   245                 250                 255
   Tyr Thr Ala Arg Asp Glu Gln Arg Ile Gly Ile Val Ala Ala Gly Tyr
               260                 265                 270
   Ala Asp Gly Tyr Pro Arg His Ala Leu Thr Gly Thr Pro Val Leu Val
   275 280 285
   Asp Gly Val Arg Thr Met Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Ala Leu Arg Val Pro
   340 345 350
   Val Val Thr Val
   355
<210> 21
   <211> 356
   <212> PRT
   <213> shigella flexneri 2a str. 301
<400> 21
   Met Thr Arg Pro Ile Gln Ala Ser Leu Asp Leu Gln Ala Leu Lys Gln
   1 5 10 15
   Asn Leu Ser Ile Val Arg Gln Ala Ala Pro His Ala Arg Val Trp Ser
   20 25 30
   Val Val Lys Ala Asn Ala Tyr Gly His Gly Ile Glu Arg Ile Trp Ser
   35 40 45
   Ala Leu Gly Ala Thr Asp Gly Phe Ala Leu Leu Asn Leu Glu Glu Ala
   50 55 60
   Ile Thr Leu Arg Glu Arg Gly Trp Lys Gly Pro Ile Leu Met Leu Glu
   65 70 75 80
   Gly Phe Phe His Ala Gln Asp Leu Glu Ile Tyr Asp Gln His Arg Leu
   85 90 95
   Thr Thr Cys Val His Ser Asn Trp Gln Leu Lys Ala Leu Gln Asn Ala
   100 105 110
   Arg Leu Lys Ala Pro Leu Asp Ile Tyr Leu Lys Val Asn Ser Gly Met
   115 120 125
   Asn Arg Leu Gly Phe Gln Ser Asp Arg Val Leu Thr Val Trp Gln Gln
   130 135 140
   Leu Arg Ala Met Ala Asn Val Gly Glu Met Thr Leu Met Ser His Phe
   145                 150                 155                 160
   Ala Glu Ala Glu His Pro Asp Gly Ile Ser Gly Ala Met Ala Arg Ile
                   165                 170                 175
   Glu Gln Ala Ala Glu Gly Leu Glu Cys Arg Arg Ser Leu Ser Asn Ser
               180                 185                 190
   Ala Ala Thr Leu Trp His Pro Glu Ala His Phe Asp Trp Val Arg Pro
           195                 200                 205
   Gly Ile Ile Leu Tyr Gly Ala Ser Pro Ser Gly Gln Trp Arg Asp Ile
       210                 215                 220
   Ala Asn Thr Gly Leu Arg Pro Val Met Thr Leu Ser Ser Glu Ile Ile
   225                 230                 235                 240
   Gly Val Gln Thr Leu Lys Ala Gly Glu Arg Val Gly Tyr Gly Gly Arg
                   245                 250                 255
   Tyr Thr Ala Arg Asp Glu Gln Arg Ile Gly Ile Val Ala Ala Gly Tyr
               260                 265                 270
   Ala Asp Gly Tyr Pro Arg His Ala Pro Thr Gly Ala Pro Val Leu Val
           275                 280                 285
   Asp Gly Val Arg Thr Met Thr Val Gly Thr Val Ser Met Asp Met Leu
       290                 295                 300
   Ala Val Asp Leu Thr Pro Cys Pro Gln Ala Gly Ile Gly Thr Pro Val
   305                 310                 315                 320
   Glu Leu Trp Gly Lys Glu Ile Lys Ile Asp Asp Val Ala Ala Ala Ala
                   325                 330                 335
   Gly Thr Val Gly Tyr Glu Leu Met Cys Ala Leu Ala Leu Arg Val Pro
               340                 345                 350
   Val Val Thr Val
           355
<210> 22
   <211> 356
   <212> PRT
   <213> Escherichia coli F11
<400> 22
   Met Thr Arg Pro Ile Gln Ala Ser Leu Asp Leu Gln Ala Leu Lys Gln
   1               5                   10                  15
   Asn Leu Ser Ile Val Arg Gln Ala Ala Pro Tyr Ala Arg Val Trp Ser
               20                  25                  30
   Val Val Lys Ala Asn Ala Tyr Gly His Gly Ile Glu Arg Ile Trp Ser
           35                  40                  45
   Ala Leu Gly Ala Thr Asp Gly Phe Ala Leu Leu Asn Leu Glu Glu Ala
       50                  55                  60
   Ile Thr Leu Arg Glu Arg Gly Trp Lys Gly Pro Ile Leu Met Leu Glu
   65                  70                  75                  80
   Gly Phe Phe His Ala Gln Asp Leu Glu Ile Tyr Asp Gln His Arg Leu
                   85                  90                  95
   Thr Thr Cys Val His Ser Asn Trp Gln Leu Lys Ala Leu Gln Asn Ala
               100                 105                 110
   Arg Leu Lys Ala Pro Leu Asp Ile Tyr Leu Lys Val Asn Ser Gly Met
           115                 120                 125
   Asn Arg Leu Gly Phe Gln Pro Asp Arg Val Leu Thr Val Trp Gln Gln
       130                 135                 140
   Leu Arg Ala Met Ala Asn Val Gly Glu Met Thr Leu Met Ser His Phe
   145                 150                 155                 160
   Ala Glu Ala Glu His Pro Asp Gly Ile Ser Gly Ala Met Ala Arg Ile
                   165                 170                 175
   Glu Gln Ala Ala Glu Gly Leu Glu Cys Arg Arg Ser Leu Ser Asn Ser
               180                 185                 190
   Ala Ala Thr Leu Trp His Pro Glu Ala His Phe Asp Trp Val Arg Pro
           195                 200                 205
   Gly Ile Ile Leu Tyr Gly Ala Ser Pro Ser Gly Gln Trp Arg Asp Ile
       210                 215                 220
   Ala Asn Thr Gly Leu Arg Pro Val Met Thr Leu Ser Ser Glu Ile Ile
   225                 230                 235                 240
   Gly Val Gln Thr Leu Lys Ala Gly Glu Arg Val Gly Tyr Gly Gly Arg
                   245                 250                 255
   Tyr Thr Ala Arg Asp Glu Gln Arg Ile Gly Ile Val Ala Ala Gly Tyr
               260                 265                 270
   Ala Asp Gly Tyr Pro Arg His Ala Pro Thr Gly Thr Pro Val Leu Val
           275                 280                 285
   Asp Gly Val Leu Thr Met Thr Val Gly Thr Val Ser Met Asp Met Leu
       290                 295                 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Ala Leu Arg Val Pro
   340 345 350
   Val Val Thr Val
   355
<210> 23
   <211> 356
   <212> PRT
   <213> Escherichia coli
<400> 23
   Met Thr Arg Pro Ile Gln Ala Ser Leu Asp Leu Gln Ala Leu Lys Gln
   1               5                   10                  15
   Asn Leu Ser Ile Val Arg Gln Ala Ala Thr His Ala Arg Val Trp Ser
               20                  25                  30
   Val Val Lys Ala Asn Ala Tyr Gly His Gly Ile Glu Arg Ile Trp Ser
           35                  40                  45
   Ala Ile Gly Ala Thr Asp Gly Phe Ala Leu Leu Asn Leu Glu Glu Ala
       50                  55                  60
   Ile Thr Leu Arg Glu Arg Gly Trp Lys Gly Pro Ile Leu Met Leu Glu
   65                  70                  75                  80
   Gly Phe Phe His Ala Gln Asp Leu Glu Ile Tyr Asp Gln His Arg Leu
                   85                  90                  95
   Thr Thr Cys Val His Ser Asn Trp Gln Leu Lys Ala Leu Gln Asn Ala
               100                 105                 110
   Arg Leu Lys Ala Pro Leu Asp Ile Tyr Leu Lys Val Asn Ser Gly Met
           115                 120                 125
   Asn Arg Leu Gly Phe Gln Pro Asp Arg Val Leu Thr Val Trp Gln Gln
       130                 135                 140
   Leu Arg Ala Met Ala Asn Val Gly Glu Met Thr Leu Met Ser His Phe
   145                 150                 155                 160
   Ala Glu Ala Glu His Pro Asp Gly Ile Ser Gly Arg Met Ala Arg Ile
                   165                 170                 175
   Glu Gln Ala Ala Glu Gly Leu Glu Cys Arg Arg Ser Leu Ser Asn Ser
   180 185 190
   Ala Ala Thr Leu Trp His Pro Glu Ala His Phe Asp Trp Val Arg Pro
   195 200 205
   Gly Ile Ile Leu Tyr Gly Arg Ser Pro Ser Gly Gln Trp Arg Asp Ile
   210 215 220
   Ala Asn Thr Gly Leu Arg Pro Val Met Thr Leu Ser Ser Glu Ile Ile
   225 230 235 240
   Gly Val Gln Thr Leu Lys Ala Gly Glu Arg Val Gly Tyr Gly Gly Arg
   245 250 255
   Tyr Thr Ala Arg Asp Glu Gln Arg Ile Gly Ile Val Ala Ala Gly Tyr
   260 265 270
   Ala Asp Gly Tyr Pro Arg His Ala Leu Thr Gly Thr Pro Val Leu Val
   275 280 285
   Asp Gly Val Arg Thr Met Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Ile Asp Asp Val Ala Ala Ala Ala
   325 330 335
   Gly Thr Val Gly Tyr Glu Leu Met Cys Ala Leu Ala Val Arg Val Pro
   340 345 350
   Val Val Thr Val
   355
<210> 24
   <211> 356
   <212> PRT
   <213> Escherichia coli CFT073
<400> 24
   Met Thr Arg Pro Ile Gln Ala Ser Leu Asp Leu Gln Ala Leu Lys Gln
   1 5 10 15
   Asn Leu Ser Ile Val Arg Gln Ala Ala Pro Tyr Ala Arg Val Trp Ser
   20 25 30
   Val Val Lys Ala Asn Ala Tyr Gly His Gly Ile Glu Arg Ile Trp Ser
   35 40 45
   Ala Leu Gly Ala Thr Asp Gly Phe Ala Leu Leu Asn Leu Glu Glu Ala
   50 55 60
   Ile Thr Leu Arg Glu Arg Gly Trp Lys Gly Pro Ile Leu Met Leu Glu
   65                  70                  75                  80
   Gly Phe Phe His Ala Gln Asp Leu Glu Ile Tyr Asp Gln His Arg Leu
                   85                  90                  95
   Thr Thr Cys Val His Ser Asn Trp Gln Leu Lys Ala Leu Gln Asn Ala
               100                 105                 110
   Arg Leu Lys Ala Pro Leu Asp Ile Tyr Leu Lys Val Asn Ser Gly Met
           115                 120                 125
   Asn Arg Leu Gly Phe Gln Pro Asp Arg Val Leu Thr Val Trp Gln Gln
       130                 135                 140
   Leu Arg Ala Met Ala Asn Val Gly Glu Met Thr Leu Met Ser His Phe
   145                 150                 155                 160
   Ala Glu Ala Glu His Pro Asp Gly Ile Ser Ser Ala Met Ala Arg Ile
                   165                 170                 175
   Glu Gln Ala Ala Glu Gly Leu Glu Cys Arg Arg Ser Leu Ala Asn Ser
               180                 185                 190
   Ala Ala Thr Leu Trp His Gln Glu Ala His Phe Asp Trp Val Arg Pro
           195                 200                 205
   Gly Ile Ile Leu Tyr Gly Ala Ser Pro Ser Gly Gln Trp Arg Asp Ile
       210                 215                 220
   Ala Asn Thr Gly Leu Arg Pro Val Met Thr Leu Ser Ser Glu Ile Ile
   225                 230                 235                 240
   Gly Val Gln Thr Leu Lys Ala Gly Glu Arg Val Gly Tyr Gly Gly Arg
                   245                 250                 255
   Tyr Thr Ala Arg Asp Glu Gln Arg Ile Gly Ile Val Ala Ala Gly Tyr
               260                 265                 270
   Ala Asp Gly Tyr Pro Arg His Ala Pro Thr Gly Thr Pro Val Leu Val
           275                 280                 285
   Asp Gly Val Leu Thr Met Thr Val Gly Thr Val Ser Met Asp Met Leu
       290                 295                 300
   Ala Val Asp Leu Thr Pro Cys Pro Gln Ala Gly Ile Gly Thr Pro Val
   305                 310                 315                 320
   Glu Leu Trp Gly Lys Glu Ile Lys Ile Asp Asp Val Ala Ala Ala Ala
                   325                 330                 335
   Gly Thr Val Gly Tyr Glu Leu Met Cys Ala Leu Ala Leu Arg Val Pro
   340 345 350
   Val Val Thr Val
   355
<210> 25
   <211> 324
   <212> PRT
   <213> Shigella boydii BS512
<400> 25
   Met Val Lys Ala Asn Ala Tyr Gly His Gly Ile Glu Arg Ile Trp Ser
   1               5                   10                  15
   Ala Leu Gly Ala Thr Asp Gly Phe Ala Leu Leu Asn Leu Glu Glu Ala
               20                  25                  30
   Ile Thr Leu Arg Glu Arg Gly Trp Lys Gly Pro Ile Leu Met Leu Glu
           35                  40                  45
   Gly Phe Phe His Ala Gln Asp Leu Glu Ile Tyr Asp Gln His Arg Leu
       50                  55                  60
   Thr Thr Cys Val His Ser Asn Trp Gln Leu Lys Ala Leu Gln Asn Ala
   65                  70                  75                  80
   Arg Leu Lys Ala Pro Leu Asp Ile Tyr Leu Lys Val Asn Ser Gly Met
                   85                  90                  95
   Asn Arg Leu Gly Phe Gln Pro Asp Arg Val Leu Thr Val Trp Gln Gln
               100                 105                 110
   Leu Arg Ala Met Ala Asn Val Gly Glu Met Thr Leu Met Ser His Phe
           115                 120                 125
   Ala Glu Ala Glu His Pro Asp Gly Ile Ser Ser Ala Met Ala Arg Ile
       130                 135                 140
   Glu Gln Ala Ala Glu Gly Leu Glu Cys Arg Arg Ser Leu Ser Asn Ser
   145                 150                 155                 160
   Ala Ala Thr Leu Trp His Pro Glu Ala His Phe Asp Trp Val Arg Pro
                   165                 170                 175
   Gly Ile Ile Leu Tyr Gly Ala Ser Pro Ser Gly Gln Trp Arg Asp Ile
               180                 185                 190
   Ala Asn Thr Gly Leu Arg Pro Val Met Thr Leu Ser Ser Glu Ile Ile
           195                 200                 205
   Gly Val Gln Thr Leu Lys Ala Gly Glu Arg Val Gly Tyr Gly Gly Arg
       210                 215                 220
   Tyr Thr Ala Arg Asp Glu Gln Arg Ile Gly Ile Val Ala Ala Gly Tyr
   225 230 235 240
   Ala Asp Gly Tyr Pro Arg His Ala Pro Thr Gly Thr Pro Val Leu Val
   245 250 255
   Asp Gly Val Arg Thr Met Thr Val Gly Thr Val Ser Met Asp Met Leu
   260 265 270
   Ala Val Asp Leu Thr Pro Cys Pro Gln Ala Gly Ile Gly Thr Pro Val
   275 280 285
   Glu Leu Trp Gly Lys Glu Ile Lys Ile Asp Asp Val Ala Ala Ala Ala
   290 295 300
   Gly Thr Val Gly Tyr Glu Leu Met Cys Ala Leu Ala Leu Arg Val Pro
   305 310 315 320
   Val Val Thr Val
<210> 26
   <211> 329
   <212> PRT
   <213> Escherichia coli 0157:H7 str. Sakai
<400> 26
   Met Thr Arg Pro Ile Gln Ala Ser Leu Asp Leu Gln Ala Leu Lys Gln
   1 5 10 15
   Asn Leu Ser Ile Val Arg Gln Ala Ala Pro His Ala Arg Val Trp Ser
   20 25 30
   Val Val Lys Ala Asn Ala Tyr Gly His Gly Ile Glu Arg Ile Trp Ser
   35 40 45
   Ala Leu Gly Ala Thr Asp Gly Phe Ala Leu Leu Asn Leu Glu Glu Ala
   50 55 60
   Ile Thr Leu Arg Glu Arg Gly Trp Lys Gly Pro Ile Leu Met Leu Glu
   65 70 75 80
   Gly Phe Phe His Ala Gln Asp Leu Glu Met Tyr Asp Gln His Arg Leu
   85 90 95
   Thr Thr Cys Val His Ser Asn Trp Gln Leu Lys Ala Leu Gln Asn Ala
   100 105 110
   Arg Leu Lys Ala Pro Leu Asp Ile Tyr Leu Lys Val Asn Ser Gly Met
   115 120 125
   Asn Arg Leu Gly Phe Gln Pro Asp Arg Val Leu Thr Val Trp Gln Gln
   130 135 140
   Leu Arg Ala Met Ala Asn Val Gly Glu Met Thr Leu Met Ser His Phe
   145 150 155 160
   Ala Glu Ala Glu His Pro Asp Gly Ile Ser Gly Ala Met Ala Arg Ile
   165 170 175
   Glu Gln Ala Ala Glu Gly Leu Glu Cys Arg Arg Ser Leu Ser Asn Ser
   180 185 190
   Ala Ala Thr Leu Trp His Pro Glu Ala His Phe Asp Trp Val Arg Pro
   195 200 205
   Gly Ile Ile Leu Tyr Gly Ala Ser Pro Ser Gly Gln Trp Arg Asp Ile
   210 215 220
   Ala Asn Thr Gly Leu Arg Pro Val Met Thr Leu Ser Ser Glu Ile Ile
   225 230 235 240
   Gly Val Gln Thr Leu Lys Ala Gly Glu Arg Val Gly Tyr Gly Gly Arg
   245 250 255
   Tyr Thr Ala Arg Asp Glu Gln Arg Ile Gly Ile Val Ala Ala Gly Tyr
   260 265 270
   Ala Asp Gly Tyr Pro Arg His Ala Pro Thr Gly Thr Pro Val Leu Val
   275 280 285
   Asp Gly Val Arg Thr Met Thr Val Gly Thr Val Ser Met Asp Met Leu
   290 295 300
   Ala Val Asp Leu Thr Pro Cys Pro Gln Arg Asp Ile Gly Thr Pro Val
   305 310 315 320
   Gly Ala Val Gly Gln Gly Asp Gln Asn
   325
<210> 27
   <211> 356
   <212> PRT
   <213> Salmonella enterica subsp. enterica serovar
<400> 27
   Met Thr Arg Pro Ile Gln Ala Ser Leu Asp Leu Gln Val Met Lys Gln
   1               5                   10                  15
   Asn Leu Ala Ile Val Arg Arg Ala Ala Pro Glu Ala Arg Val Trp Ser
               20                  25                  30
   Val Val Lys Ala Asn Ala Tyr Gly His Gly Ile Glu Arg Val Trp Ser
           35                  40                  45
   Ala Leu Gly Ala Thr Asp Gly Phe Ala Met Leu Asn Leu Glu Glu Ala
   50 55 60
   Ile Thr Leu Arg Glu Arg Gly Trp Lys Gly Pro Ile Leu Met Leu Glu
   65 70 75 80
   Gly Phe Phe His Ala Gln Asp Leu Glu Ala Tyr Asp Thr Tyr Arg Leu
   85 90 95
   Thr Thr Cys Ile His Ser Asn Trp Gln Leu Lys Ala Leu Gln Asn Ala
   100 105 110
   Arg Leu Asn Ala Pro Leu Asp Ile Tyr Val Lys Val Asn Ser Gly Met
   115 120 125
   Asn Arg Leu Gly Phe Gln Pro Glu Arg Ala Gln Thr Val Trp Gln Gln
   130 135 140
   Leu Arg Ala Met Arg Asn Val Gly Glu Met Thr Leu Met Ser His Phe
   145 150 155 160
   Ala Gln Ala Asp His Pro Glu Gly Ile Gly Glu Ala Met Arg Arg Ile
   165 170 175
   Ala Leu Ala Thr Glu Gly Leu Gln Cys Ala Tyr Ser Leu Ser Asn Ser
   180 185 190
   Ala Ala Thr Leu Trp His Pro Gln Ala His Tyr Asp Trp Val Arg Pro
   195 200 205
   Gly Ile Ile Leu Tyr Gly Ala Ser Pro Ser Gly Gln Trp Arg Asp Ile
   210 215 220
   Ala Asp Thr Gly Leu Lys Pro Val Met Thr Leu Ser Ser Glu Ile Ile
   225 230 235 240
   Gly Val Gln Thr Leu Ser Val Gly Glu Arg Val Gly Tyr Gly Gly Arg
   245 250 255
   Tyr Ser Val Thr Gln Glu Gln Arg Ile Gly Ile Val Ala Ala Gly Tyr
   260 265 270
   Ala Asp Gly Tyr Pro Arg His Ala Pro Thr Gly Thr Pro Val Leu Val
   275 280 285
   Asp Gly Ile Arg Thr Arg Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Val Asp Asp Val Ala Ser Ala Ala
                   325                 330                 335
   Gly Thr Leu Gly Tyr Glu Leu Leu Cys Ala Val Ala Pro Arg Val Pro
               340                 345                 350
   Phe Val Thr Thr
           355
<210> 28
   <211> 356
   <212> PRT
   <213> Salmonella typhimurium LT2
<400> 28
   Met Thr Arg Pro Ile Gln Ala Ser Leu Asp Leu Gln Val Met Lys Gln
   1               5                   10                  15
   Asn Leu Ala Ile Val Arg Arg Ala Ala Pro Glu Ala Arg Val Trp Ser
               20                  25                  30
   Val Val Lys Ala Asn Ala Tyr Gly His Gly Ile Glu Arg Val Trp Ser
           35                  40                  45
   Ala Leu Gly Ala Thr Asp Gly Phe Ala Met Leu Asn Leu Glu Glu Ala
       50                  55                  60
   Ile Thr Leu Arg Glu Arg Gly Trp Lys Gly Pro Ile Leu Met Leu Glu
   65                  70                  75                  80
   Gly Phe Phe His Ala Gln Asp Leu Glu Ala Tyr Asp Thr Tyr Arg Leu
                   85                  90                  95
   Thr Thr Cys Ile His Ser Asn Trp Gln Leu Lys Ala Leu Gln Asn Ala
               100                 105                 110
   Arg Leu Asn Ala Pro Leu Asp Ile Tyr Val Lys Val Asn Ser Gly Met
           115                 120                 125
   Asn Arg Leu Gly Phe Gln Pro Glu Arg Ala Gln Thr Val Trp Gln Gln
       130                 135                 140
   Leu Arg Ala Met Arg Asn Val Gly Glu Met Thr Leu Met Ser His Phe
   145                 150                 155                 160
   Ala Gln Ala Asp His Pro Glu Gly Ile Gly Glu Ala Met Arg Arg Ile
                   165                 170                 175
   Ala Leu Ala Thr Glu Gly Leu Gln Cys Ala Tyr Ser Leu Ser Asn Ser
               180                 185                 190
   Ala Ala Thr Leu Trp His Pro Gln Ala His Tyr Asp Trp Val Arg Pro
   195 200 205
   Gly Ile Ile Leu Tyr Gly Ala Ser Pro Ser Gly Gln Trp Arg Asp Ile
   210 215 220
   Ala Asp Thr Gly Leu Lys Pro Val Met Thr Leu Ser Ser Glu Ile Ile
   225 230 235 240
   Gly Val Gln Thr Leu Ser Ala Gly Glu Arg Val Gly Tyr Gly Gly Gly
   245 250 255
   Tyr Ser Val Thr Gln Glu Gln Arg Ile Gly Ile Val Ala Ala Gly Tyr
   260 265 270
   Ala Asp Gly Tyr Pro Arg His Ala Pro Thr Gly Thr Pro Val Leu Val
   275 280 285
   Asp Gly Ile Arg Thr Arg Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Val Asp Asp Val Ala Ser Ala Ala
   325 330 335
   Gly Thr Leu Gly Tyr Glu Leu Leu Cys Ala Val Ala Pro Arg Val Pro
   340 345 350
   Phe Val Thr Thr
   355
<210> 29
   <211> 356
   <212> PRT
   <213> Salmonella enterica subsp. enterica serovar Typhi
<400> 29
   Met Thr Arg Pro Ile Gln Ala Ser Leu Asp Leu Gln Val Met Lys Gln
   1 5 10 15
   Asn Leu Ala Ile Val Arg Arg Ala Ala Pro Glu Ala Arg Val Trp Ser
   20 25 30
   Val Val Lys Ala Asn Ala Tyr Gly His Gly Ile Glu Arg Val Trp Ser
   35 40 45
   Ala Leu Gly Ala Thr Asp Gly Phe Ala Met Leu Asn Leu Glu Glu Ala
   50 55 60
   Ile Thr Leu Arg Glu Arg Gly Trp Lys Gly Pro Ile Leu Met Leu Glu
   65 70 75 80
   Gly Phe Phe His Ala Gln Asp Leu Glu Ala Tyr Asp Thr Tyr Arg Leu
   85 90 95
   Thr Thr Cys Ile His Ser Asn Trp Gln Leu Lys Ala Leu Gln Asn Ala
   100 105 110
   Arg Leu Asn Ala Pro Leu Asp Ile Tyr Val Lys Val Asn Ser Gly Met
   115 120 125
   Asn Arg Leu Gly Phe Gln Pro Glu Arg Ala Gln Thr Val Trp Gln Gln
   130 135 140
   Leu Arg Ala Met Arg Asn Val Gly Glu Met Thr Leu Met Ser His Phe
   145 150 155 160
   Ala Gln Ala Asp His Pro Glu Gly Ile Gly Glu Ala Met Arg Arg Ile
   165 170 175
   Ala Leu Ala Thr Glu Gly Leu Gln Cys Ala Tyr Ser Leu Ser Asn Ser
   180 185 190
   Ala Ala Thr Leu Trp His Pro Gln Ala His Tyr Asp Trp Val Arg Pro
   195 200 205
   Gly Ile Ile Leu Tyr Gly Ala Ser Pro Ser Gly Gln Trp Arg Asp Ile
   210 215 220
   Ala Asp Thr Gly Leu Lys Pro Val Met Thr Leu Ser Ser Glu Ile Ile
   225 230 235 240
   Gly Val Gln Thr Leu Ser Ala Gly Glu Arg Val Gly Tyr Gly Gly Gly
   245 250 255
   Tyr Ser Val Thr Gln Glu Gln Arg Ile Gly Ile Val Ala Ala Gly Tyr
   260 265 270
   Ala Asp Gly Tyr Pro Arg His Ala Pro Thr Gly Thr Pro Val Leu Val
   275 280 285
   Asp Gly Ile Arg Thr Arg Thr Val Gly Thr Val Ser Met Asp Met Leu
   290 295 300
   Ala Val Asp Leu Thr Pro Cys Pro Gln Ala Gly Ile Gly Thr Pro Val
   305 310 315 320
   Glu Leu Trp Gly Lys Glu Ile Lys Val Asp Asp Val Ala Ser Ala Ala
   325 330 335
   Gly Thr Leu Gly Tyr Gly Leu Leu Cys Ala Val Ala Pro Arg Val Pro
   340 345 350
   Phe Val Thr Thr
   355
<210> 30
   <211> 356
   <212> PRT
   <213> Enterobacter sp. 638
<400> 30
   Met Ser Arg Pro Ile Leu Ala Gln Leu Asp Leu Gln Ala Leu Lys Asn
   1               5                   10                  15
   Asn Leu Gln Ile Val Arg Arg Ala Ala Pro Gly Ser Arg Val Trp Ser
               20                  25                  30
   Val Val Lys Ala Asn Gly Tyr Gly His Gly Ile Glu Ser Ile Trp Ser
           35                  40                  45
   Ala Leu Ser Ala Thr Asp Gly Phe Ala Leu Leu Asn Leu Glu Glu Ala
       50                  55                  60
   Ile Leu Leu Arg Glu Arg Gly Trp Lys Gly Pro Ile Leu Leu Leu Glu
   65                  70                  75                  80
   Gly Phe Phe His Ala Asp Glu Leu Pro Leu Leu Asp Lys Tyr Arg Leu
                   85                  90                  95
   Thr Thr Ser Val His Ser Asn Trp Gln Ile Lys Ala Leu Gln Asn Ala
               100                 105                 110
   Lys Leu His Ser Pro Leu Asp Ile Tyr Leu Lys Met Asn Ser Gly Met
           115                 120                 125
   Asn Arg Leu Gly Phe Gln Pro Glu Arg Val Asn Thr Val Trp Gln Gln
       130                 135                 140
   Leu Arg Ala Ile Lys Asn Val Gly Glu Met Thr Leu Met Ala His Phe
   145                 150                 155                 160
   Ala Asp Ala Glu Lys Pro Asp Gly Ile Thr Asn Ala Met Ala Arg Ile
                   165                 170                 175
   Glu Gln Ala Ala Glu Gly Leu Asp Cys Ala Arg Ser Leu Ser Asn Ser
               180                 185                 190
   Ala Ala Thr Leu Trp His Pro Glu Ala His Tyr Gln Trp Val Arg Pro
           195                 200                 205
   Gly Ile Val Leu Tyr Gly Ala Ser Pro Ser Gly Gln Trp Gln Asp Ile
       210                 215                 220
   Ala Asn Ser Gly Leu Lys Pro Val Met Thr Leu Arg Ser Glu Ile Ile
   225                 230                 235                 240
   Gly Ile Gln Thr Leu Lys Ala Gly Asp Thr Val Gly Tyr Gly Ser Arg
   245 250 255
   Tyr Arg Ser Thr Gly Glu Gln Arg Ile Gly Ile Val Ala Gly Gly Tyr
   260 265 270
   Ala Asp Gly Tyr Pro Arg Leu Ala Pro Ser Gly Thr Pro Val Trp Ile
   275 280 285
   Asp Gly Val Arg Thr Gly Thr Val Gly Thr Ile Ser Met Asp Met Met
   290 295 300
   Ala Ile Asp Leu Thr Pro Cys Pro Gln Ala Gly Ile Gly Ser Pro Val
   305 310 315 320
   Glu Leu Trp Gly Asn Glu Val Lys Val Asp Asp Val Ala Ser Ala Ala
   325 330 335
   Gly Thr Val Gly Tyr Glu Leu Leu Thr Ala Leu Ala Pro Arg Val Pro
   340 345 350
   Val Val Thr Val
   355
<210> 31
   <211> 359
   <212> PRT
   <213> Artificial
<220>
   <223> synthetic gene
<400> 31
   Met Gln Ala Ala Thr Val Val Ile Asn Arg Arg Ala Leu Arg His Asn
   1 5 10 15
   Leu Gln Arg Leu Arg Glu Leu Ala Pro Ala Ser Lys Met Val Ala Val
   20 25 30
   Val Lys Ala Asn Ala Tyr Gly His Gly Leu Leu Glu Thr Ala Arg Thr
   35 40 45
   Leu Pro Asp Ala Asp Ala Phe Gly Val Ala Arg Leu Glu Glu Ala Leu
   50 55 60
   Arg Leu Arg Ala Gly Gly Ile Thr Lys Pro Val Leu Leu Leu Glu Gly
   65 70 75 80
   Phe Phe Asp Ala Arg Asp Leu Pro Thr Ile Ser Ala Gln His Phe His
   85 90 95
   Thr Ala Val His Asn Glu Gly Gln Leu Ala Ala Leu Glu Glu Ala Ser
   100 105 110
   Leu Asp Glu Pro Val Thr Val Trp Met Lys Leu Asp Thr Gly Met His
   115 120 125
   Arg Leu Gly Val Arg Pro Glu Gln Ala Glu Ala Phe Tyr His Arg Leu
   130 135 140
   Thr Gln Cys Lys Asn Val Arg Gln Pro Val Asn Ile Val Ser His Phe
   145 150 155 160
   Ala Arg Ala Asp Glu Pro Lys Cys Gly Ala Thr Glu Lys Gln Leu Ala
   165 170 175
   Ile Phe Asn Thr Phe Cys Glu Gly Lys Pro Gly Gln Arg Ser Ile Ala
   180 185 190
   Ala Ser Gly Gly Ile Leu Leu Trp Pro Gln Ser His Phe Asp Trp Val
   195 200 205
   Arg Pro Gly Ile Ile Leu Tyr Gly Val Ser Pro Leu Glu Asp Arg Ser
   210 215 220
   Thr Gly Ala Asp Phe Gly Cys Gln Pro Val Met Ser Leu Thr Ser Ser
   225 230 235 240
   Leu Ile Ala Val Arg Glu His Lys Ala Gly Glu Pro Val Gly Tyr Gly
   245 250 255
   Gly Thr Trp Val Ser Glu Arg Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Pro Ile Val Gly Arg Val Ala Met Asp
   290 295 300
   Met Ile Cys Val Asp Leu Gly Pro Gln Ala Gln Asp Lys Ala Gly Asp
   305 310 315 320
   Pro Val Ile Leu Trp Gly Glu Gly Leu Pro Val Glu Arg Val Ala Glu
   325 330 335
   Met Thr Lys Val Ser Ala Tyr Glu Leu Ile Thr Arg Leu Thr Ser Arg
   340 345 350
   Val Ala Met Lys Tyr Val Asp
   355
<210> 32
   <211> 359
   <212> PRT
   <213> Escherichia coli 0157:H7 EDL933
<400> 32
   Met Gln Ala Ala Thr Val Val Ile Asn Arg Arg Ala Leu Arg His Asn
   1               5                   10                  15
   Leu Gln Arg Leu Arg Glu Leu Ala Pro Ala Ser Lys Met Val Ala Val
               20                  25                  30
   Val Lys Ala Asn Ala Tyr Gly His Gly Leu Leu Glu Thr Ala Arg Thr
           35                  40                  45
   Leu Pro Asp Ala Asp Ala Phe Gly Val Ala Arg Leu Glu Glu Ala Leu
       50                  55                  60
   Arg Leu Arg Ala Gly Gly Ile Thr Lys Pro Val Leu Leu Leu Glu Gly
   65                  70                  75                  80
   Phe Phe Asp Ala Arg Asp Leu Pro Thr Ile Ser Ala Gln His Phe His
                   85                  90                  95
   Thr Ala Val His Asn Glu Glu Gln Leu Ala Ala Leu Glu Glu Ala Ser
               100                 105                 110
   Leu Asp Glu Pro Val Thr Val Trp Met Lys Leu Asp Thr Gly Met His
           115                 120                 125
   Arg Leu Gly Val Arg Pro Glu Gln Ala Glu Ala Phe Tyr His Arg Leu
       130                 135                 140
   Thr Gln Cys Lys Asn Val Arg Gln Pro Val Asn Ile Val Ser His Phe
   145                 150                 155                 160
   Ala Arg Ala Asp Glu Pro Lys Cys Gly Ala Thr Glu Lys Gln Leu Ala
                   165                 170                 175
   Ile Phe Asn Thr Phe Cys Glu Gly Lys Pro Gly Gln Arg Ser Ile Ala
               180                 185                 190
   Ala Ser Gly Gly Ile Leu Leu Trp Pro Gln Ser His Phe Asp Trp Val
           195                 200                 205
   Arg Pro Gly Ile Ile Leu Tyr Gly Val Ser Pro Leu Glu Asp Arg Ser
       210                 215                 220
   Thr Gly Ala Asp Phe Gly Cys Gln Pro Val Met Ser Leu Thr Ser Ser
   225                 230                 235                 240
   Leu Ile Ala Val Arg Glu His Lys Val Gly Glu Pro Val Gly Tyr Gly
                   245                 250                 255
   Gly Thr Trp Ile Ser Glu Arg Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Pro Ile Val Gly Arg Val Ala Met Asp
   290 295 300
   Met Ile Cys Val Asp Leu Gly Pro Gln Ala Gln Asp Lys Ala Gly Asp
   305 310 315 320
   Pro Val Ile Leu Trp Gly Glu Gly Leu Pro Val Glu Arg Ile Ala Glu
   325 330 335
   Met Thr Lys Val Ser Ala Tyr Glu Leu Ile Thr Arg Leu Thr Ser Arg
   340 345 350
   Val Ala Met Lys Tyr Val Asp
   355
<210> 33
   <211> 359
   <212> PRT
   <213> Shigella sonnei Ss046
<400> 33
   Met Gln Ala Ala Thr Val Val Ile Asn Arg Arg Ala Leu Arg His Asn
   1               5                   10                  15
   Leu Gln Arg Leu Arg Glu Leu Ala Pro Ala Ser Lys Met Val Ala Val
               20                  25                  30
   Val Lys Ala Asn Ala Tyr Gly His Gly Leu Leu Glu Thr Ala Arg Thr
           35                  40                  45
   Leu Pro Asp Ala Asp Ala Phe Gly Val Ala Arg Leu Glu Glu Ala Leu
       50                  55                  60
   Arg Leu Arg Ala Gly Gly Ile Thr Lys Pro Val Leu Leu Leu Glu Gly
   65                  70                  75                  80
   Phe Phe Asp Ala Arg Asp Leu Pro Thr Ile Ser Ala Gln His Phe His
                   85                  90                  95
   Thr Ala Val His Asn Glu Glu Gln Leu Ala Ala Leu Glu Glu Ala Ser
               100                 105                 110
   Leu Asp Glu Pro Val Thr Val Trp Met Lys Leu Asp Thr Gly Met His
           115                 120                 125
   Arg Leu Gly Val Arg Pro Glu Gln Ala Glu Ala Phe Tyr His Arg Leu
       130                 135                 140
   Thr Gln Cys Lys Asn Val Arg Gln Pro Val Asn Ile Val Ser His Phe
   145 150 155 160
   Ala Arg Ala Asp Glu Pro Lys Cys Gly Ala Thr Glu Lys Gln Leu Ala
   165 170 175
   Ile Phe Asn Thr Phe Cys Glu Gly Lys Pro Gly Gln Arg Ser Ile Ala
   180 185 190
   Ala Ser Gly Gly Ile Leu Leu Trp Pro Gln Ser His Phe Asp Trp Val
   195 200 205
   Arg Pro Gly Ile Ile Leu Tyr Gly Val Ser Pro Leu Glu Asp Arg Ser
   210 215 220
   Ile Gly Ala Asp Phe Gly Cys Gln Pro Val Met Ser Leu Thr Ser Ser
   225 230 235 240
   Leu Ile Ala Val Arg Glu His Lys Ala Gly Glu Pro Val Gly Tyr Gly
   245 250 255
   Gly Thr Trp Val Ser Glu Arg Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Pro Ile Val Gly Arg Val Ala Met Asp
   290 295 300
   Met Ile Cys Val Asp Leu Gly Pro Gln Ala Gln Asp Lys Ala Gly Asp
   305 310 315 320
   Pro Val Ile Leu Trp Gly Glu Gly Leu Pro Val Glu Arg Ile Ala Glu
   325 330 335
   Met Thr Lys Val Ser Ala Tyr Glu Leu Ile Thr Arg Leu Thr Ser Arg
   340 345 350
   Val Ala Met Lys Tyr Val Asp
   355
<210> 34
   <211> 359
   <212> PRT
   <213> Escherichia coli E110019
<400> 34
   Met Gln Ala Ala Thr Val Val Ile Asn Arg Arg Ala Leu Arg His Asn
   1 5 10 15
   Leu Gln Arg Leu Arg Glu Leu Ala Pro Ala Ser Lys Met Val Ala Val
   20 25 30
   Val Lys Ala Asn Ala Tyr Gly His Gly Leu Leu Glu Thr Ala Arg Thr
   35 40 45
   Leu Pro Asp Ala Asp Ala Phe Gly Val Ala Arg Leu Glu Glu Ala Leu
   50 55 60
   Arg Leu Arg Ala Gly Gly Ile Thr Lys Pro Val Leu Leu Leu Glu Gly
   65 70 75 80
   Phe Phe Asp Ala Arg Asp Leu Pro Met Ile Ser Ala Gln His Phe His
   85 90 95
   Thr Ala Val His Asn Glu Glu Gln Leu Ala Ala Leu Glu Glu Ala Ser
   100 105 110
   Leu Asp Glu Pro Val Thr Val Trp Met Lys Leu Asp Thr Gly Met His
   115 120 125
   Arg Leu Gly Val Arg Pro Glu Gln Ala Glu Ala Phe Tyr His Arg Leu
   130 135 140
   Thr Gln Cys Lys Asn Val Arg Gln Pro Val Asn Ile Val Ser His Phe
   145 150 155 160
   Ala Arg Ala Asp Glu Pro Lys Cys Gly Ala Thr Glu Lys Gln Leu Ala
   165 170 175
   Ile Phe Asn Thr Phe Cys Glu Gly Lys Pro Gly Gln Arg Ser Ile Ala
   180 185 190
   Ala Ser Gly Gly Ile Leu Leu Trp Pro Gln Ser His Phe Asp Trp Val
   195 200 205
   Arg Pro Gly Ile Ile Leu Tyr Gly Val Ser Pro Leu Glu Asp Arg Ser
   210 215 220
   Thr Gly Ala Asp Phe Gly Cys Gln Pro Val Met Ser Leu Thr Ser Ser
   225 230 235 240
   Leu Ile Ala Val Arg Glu His Lys Ala Gly Glu Pro Val Gly Tyr Gly
   245 250 255
   Gly Thr Trp Val Ser Glu Arg Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Pro Ile Val Gly Arg Val Ala Met Asp
   290 295 300
   Met Ile Cys Val Asp Leu Gly Pro Gln Ala Gln Asp Lys Ala Gly Asp
   305 310 315 320
   Pro Val Ile Leu Trp Gly Glu Gly Leu Pro Val Glu Arg Ile Ala Glu
   325 330 335
   Met Thr Lys Val Ser Ala Tyr Glu Leu Ile Thr Arg Leu Thr Ser Arg
   340 345 350
   Val Ala Met Lys Tyr Val Asp
   355
<210> 35
   <211> 359
   <212> PRT
   <213> Escherichia coli B171
<400> 35
   Met Gln Ala Ala Thr Val Val Ile Asn Arg Arg Ala Leu Arg His Asn
   1 5 10 15
   Leu Gln Arg Leu Arg Glu Leu Ala Pro Ala Ser Lys Met Val Ala Val
   20 25 30
   Val Lys Ala Asn Ala Tyr Gly His Gly Leu Leu Glu Thr Ala Arg Thr
   35 40 45
   Leu Pro Asp Ala Asp Ala Phe Gly Val Ala Arg Leu Glu Glu Ala Leu
   50 55 60
   Arg Leu Arg Ala Gly Gly Ile Ile Lys Pro Val Leu Leu Leu Glu Gly
   65 70 75 80
   Phe Phe Asp Ala Arg Asp Leu Pro Thr Ile Ser Ala Gln His Phe His
   85 90 95
   Thr Ala Val His Asn Glu Glu Gln Leu Ala Ala Leu Glu Glu Ala Ser
   100 105 110
   Leu Asp Glu Pro Val Thr Val Trp Met Lys Leu Asp Thr Gly Met His
   115 120 125
   Arg Leu Gly Val Arg Pro Glu Gln Ala Glu Ala Phe Tyr His Arg Leu
   130 135 140
   Thr Gln Cys Lys Asn Val Arg Gln Pro Val Asn Ile Val Ser His Phe
   145 150 155 160
   Ala Arg Ala Asp Glu Pro Lys Cys Gly Ala Thr Glu Lys Gln Leu Ala
   165 170 175
   Ile Phe Asn Thr Phe Cys Glu Gly Lys Pro Gly Gln Arg Ser Ile Ala
   180 185 190
   Ala Ser Gly Gly Ile Leu Leu Trp Pro Gln Ser His Phe Asp Trp Val
   195 200 205
   Arg Pro Gly Ile Ile Leu Tyr Gly Val Ser Pro Leu Glu Asp Arg Ser
   210 215 220
   Thr Gly Ala Asp Phe Gly Cys Gln Pro Val Met Ser Leu Thr Ser Ser
   225 230 235 240
   Leu Ile Ala Val Arg Glu His Lys Ala Gly Glu Pro Val Gly Tyr Gly
   245 250 255
   Gly Thr Trp Val Ser Glu Arg Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Pro Ile Val Gly Arg Val Ala Met Asp
   290 295 300
   Met Ile Cys Val Asp Leu Gly Pro Gln Ala Gln Asp Lys Ala Gly Asp
   305 310 315 320
   Pro Val Ile Leu Trp Gly Glu Gly Leu Pro Val Glu Arg Ile Ala Glu
   325 330 335
   Met Thr Lys Val Ser Ala Tyr Glu Leu Ile Thr Arg Leu Thr Ser Arg
   340 345 350
   Val Ala Met Lys Tyr Val Asp
   355
<210> 36
   <211> 359
   <212> PRT
   <213> shigella boydii BS512
<400> 36
   Met Gln Ala Ala Thr Val Val Ile Asn Arg Arg Ala Leu Arg His Asn
   1               5                   10                  15
   Leu Gln Arg Leu Arg Glu Leu Ala Pro Ala Ser Lys Met Val Ala Val
               20                  25                  30
   Val Lys Ala Asn Ala Tyr Gly His Gly Leu Leu Glu Thr Ala Arg Thr
           35                  40                  45
   Leu Pro Asp Ala Asp Ala Phe Gly Val Ala Arg Leu Glu Glu Ala Leu
           50                  55                  60
   Arg Leu Arg Ala Gly Gly Ile Thr Lys Pro Val Leu Leu Leu Glu Gly
   65                  70                  75                  80
   Phe Phe Asp Ala Arg Asp Leu Pro Thr Ile Ser Ala Gln His Phe His
                   85                  90                  95
   Thr Ala Val His Asn Glu Glu Gln Leu Ala Ala Leu Glu Glu Ala Ser
               100                 105                 110
   Leu Asp Glu Pro Val Thr Val Trp Met Lys Leu Asp Thr Gly Met His
           115                 120                 125
   Arg Leu Gly Val Arg Pro Glu Gln Ala Gly Ala Phe Tyr His Arg Leu
       130                 135                 140
   Thr Gln Cys Lys Asn Val Arg Gln Pro Val Asn Ile Val Ser His Phe
   145                 150                 155                 160
   Ala Arg Ala Asp Glu Pro Lys Cys Gly Ala Thr Glu Lys Gln Leu Ala
                   165                 170                 175
   Ile Phe Asn Thr Phe Cys Glu Gly Lys Pro Gly Gln Arg Ser Ile Ala
               180                 185                 190
   Ala Ser Gly Gly Ile Leu Leu Trp Pro Gln Ser His Phe Asp Trp Val
           195                 200                 205
   Arg Pro Gly Ile Ile Leu Tyr Gly Val Ser Pro Leu Glu Asp Arg Ser
       210                 215                 220
   Thr Gly Ala Asp Phe Gly Cys Gln Pro Val Met Ser Leu Thr Ser Ser
   225                 230                 235                 240
   Leu Ile Ala Val Arg Glu His Lys Ala Gly Glu Pro Val Gly Tyr Gly
                   245                 250                 255
   Gly Thr Trp Val Ser Glu Arg Asp Thr Arg Leu Gly Val Val Ala Met
               260                 265                 270
   Gly Tyr Gly Asp Gly Tyr Pro Arg Ala Ala Pro Ser Gly Thr Pro Val
           275                 280                 285
   Leu Val Asn Gly Arg Glu Val Pro Ile Val Gly Arg Val Ala Met Asp
       290                 295                 300
   Met Ile Cys Val Asp Leu Gly Pro Gln Ala Gln Asp Lys Ala Gly Asp
   305                 310                 315                 320
   Pro Val Ile Leu Trp Gly Glu Gly Leu Pro Val Glu Arg Ile Ala Glu
                   325                 330                 335
   Met Thr Lys Val Ser Ala Tyr Glu Leu Ile Thr Arg Leu Thr Ser Arg
   340 345 350
   Val Ala Met Lys Tyr Val Asp
   355
<210> 37
   <211> 359
   <212> PRT
   <213> Escherichia coli E24377A
<400> 37
   Met Gln Ala Ala Thr Val Val Ile Asn Arg Arg Ala Leu Arg His Asn
   1 5 10 15
   Leu Gln Arg Leu Arg Glu Leu Ala Pro Ala Ser Lys Met Val Ala Val
   20 25 30
   Val Lys Ala Asn Ala Tyr Gly His Gly Leu Leu Glu Thr Ala Arg Thr
   35 40 45
   Leu Pro Asp Ala Asp Ala Phe Gly Val Ala Arg Leu Glu Glu Ala Leu
   50 55 60
   Arg Leu Arg Ala Gly Gly Ile Thr Lys Pro Val Leu Leu Leu Glu Gly
   65 70 75 80
   Phe Phe Asp Ala Arg Asp Leu Pro Thr Ile Ser Ala Gln His Phe His
   85 90 95
   Thr Ala Val His Asn Glu Glu Gln Leu Ala Ala Leu Glu Glu Ala Ser
   100 105 110
   Leu Asp Glu Pro Val Thr Val Trp Met Lys Leu Asp Thr Gly Met His
   115 120 125
   Arg Leu Gly Val Arg Pro Glu Gln Ala Glu Ala Phe Tyr His Arg Leu
   130 135 140
   Thr Gln Cys Lys Asn Val Arg Gln Pro Val Asn Ile Val Ser His Phe
   145 150 155 160
   Ala Arg Ala Asp Glu Pro Lys Cys Gly Ala Thr Glu Lys Gln Leu Ala
   165 170 175
   Ile Phe Asn Thr Phe Cys Glu Gly Lys Ser Gly Gln Arg Ser Ile Ala
   180 185 190
   Ala Ser Gly Gly Ile Leu Leu Trp Pro Gln Ser His Phe Asp Trp Val
   195 200 205
   Arg Pro Gly Ile Ile Leu Tyr Gly Val Ser Pro Leu Glu Asp Arg Ser
       210                 215                 220
   Thr Gly Ala Asp Phe Gly Cys Gln Pro Val Met Ser Leu Thr Ser Ser
   225                 230                 235                 240
   Leu Ile Ala Val Arg Glu His Lys Ala Gly Glu Pro Val Gly Tyr Gly
                   245                 250                 255
   Gly Thr Trp Val Ser Glu Arg Asp Thr Arg Leu Gly Val Val Ala Met
               260                 265                 270
   Gly Tyr Gly Asp Gly Tyr Pro Arg Ala Ala Pro Ser Gly Thr Pro Val
           275                 280                 285
   Leu Val Asn Gly Arg Glu Val Pro Ile Val Gly Arg Val Ala Met Asp
       290                 295                 300
   Met Ile Cys Val Asp Leu Gly Pro Gln Ala Gln Asp Lys Ala Gly Asp
   305                 310                 315                 320
   Pro Val Ile Leu Trp Gly Glu Gly Leu Pro Val Glu Arg Ile Ala Glu
                   325                 330                 335
   Met Thr Lys Val Ser Ala Tyr Glu Leu Ile Thr Arg Leu Thr Ser Arg
               340                 345                 350
   Val Ala Met Lys Tyr Val Asp
           355
<210> 38
   <211> 359
   <212> PRT
   <213> shigella dysenteriae sd197
<400> 38
   Met Gln Ala Ala Thr Val Val Ile Asn Arg Arg Ala Leu Arg His Asn
   1               5                   10                  15
   Leu Gln Arg Leu Arg Glu Leu Ala Pro Ala Ser Lys Met Val Ala Val
               20                  25                  30
   Val Lys Ala Asn Ala Tyr Gly His Gly Leu Leu Glu Thr Ala Arg Thr
           35                  40                  45
   Leu Pro Asp Ala Asp Ala Phe Gly Val Ala Arg Leu Glu Glu Ala Leu
       50                  55                  60
   Arg Leu Arg Ala Gly Gly Ile Thr Lys Pro Val Leu Leu Leu Glu Gly
   65                  70                  75                  80
   Phe Phe Asp Ala Arg Asp Leu Pro Thr Ile Ser Ala Gln His Phe His
                   85                  90                  95
   Thr Ala Val His Asn Glu Glu Gln Leu Ala Ala Leu Glu Glu Ala Ser
               100                 105                 110
   Leu Asp Glu Pro Val Thr Val Trp Met Lys Leu Asp Thr Gly Met His
           115                 120                 125
   Arg Leu Gly Val Arg Pro Glu Gln Ala Glu Ala Phe Tyr His Arg Leu
       130                 135                 140
   Thr Gln Cys Lys Asn Val Arg Gln Pro Val Asn Ile Val Ser His Phe
   145                 150                 155                 160
   Ala Arg Ala Asp Glu Pro Lys Cys Gly Ala Thr Glu Lys Gln Leu Ala
                   165                 170                 175
   Ile Phe Asn Thr Phe Cys Glu Gly Lys Pro Gly Gln Arg Ser Ile Ala
               180                 185                 190
   Ala Ser Gly Gly Ile Leu Leu Trp Pro Gln Ser His Phe Asp Trp Val
           195                 200                 205
   Arg Pro Gly Ile Ile Leu Tyr Gly Val Ser Pro Leu Glu Asp Arg Ser
       210                 215                 220
   Thr Gly Ala Asp Phe Gly Cys Gln Pro Val Met Ser Leu Thr Ser Ser
   225                 230                 235                 240
   Leu Ile Ala Val Arg Glu His Lys Val Gly Glu Pro Val Gly Tyr Gly
                   245                 250                 255
   Gly Thr Trp Ile Ser Glu Arg Asp Thr Arg Leu Gly Val Val Ala Met
               260                 265                 270
   Gly Tyr Gly Asp Gly Tyr Pro Arg Ala Ala Pro Ser Gly Thr Pro Val
           275                 280                 285
   Leu Val Asn Gly Arg Glu Val Pro Ile Val Gly Arg Val Ala Met Asp
       290                 295                 300
   Met Ile Cys Val Asp Leu Gly Pro Gln Ala Gln Asp Lys Ala Gly Asp
   305                 310                 315                 320
   Pro Val Ile Leu Trp Gly Glu Gly Leu Pro Val Glu Arg Ile Ala Glu
                   325                 330                 335
   Met Thr Lys Val Ser Ala Tyr Glu Leu Ile Ala Arg Leu Thr Ser Arg
               340                 345                 350
   Val Ala Met Lys Tyr Val Asp
           355
<210> 39
   <211> 359
   <212> PRT
   <213> Artificial
<220>
   <223> synthetic gene
<400> 39
   Met Gln Val Ala Thr Val Val Ile Asn Arg Arg Ala Leu Arg His Asn
   1               5                   10                  15
   Leu Gln Arg Leu Arg Glu Leu Ala Pro Ala Ser Lys Leu Val Ala Val
               20                  25                  30
   Val Lys Ala Asn Ala Tyr Gly His Gly Leu Leu Glu Thr Ala Arg Thr
           35                  40                  45
   Leu Pro Asp Ala Asp Ala Phe Gly Val Ala Arg Leu Glu Glu Ala Leu
       50                  55                  60
   Arg Leu Arg Ala Gly Gly Ile Thr Lys Pro Val Leu Leu Leu Glu Gly
   65                  70                  75                  80
   Phe Phe Asp Ala Arg Asp Leu Pro Thr Ile Ser Ala Gln His Phe His
                   85                  90                  95
   Thr Ala Val His Asn Glu Gly Gln Leu Ala Ala Leu Glu Glu Ala Ser
               100                 105                 110
   Leu Asp Glu Pro Val Thr Val Trp Met Lys Leu Asp Thr Gly Met His
           115                 120                 125
   Arg Leu Gly Val Arg Pro Glu Gln Ala Glu Ala Phe Tyr His Arg Leu
       130                 135                 140
   Thr Gln Cys Lys Asn Val Arg Gln Pro Val Asn Ile Val Ser His Phe
   145                 150                 155                 160
   Ala Arg Ala Asp Glu Pro Lys Cys Gly Ala Thr Glu Lys Gln Leu Ala
                   165                 170                 175
   Ile Phe Asn Thr Phe Cys Glu Gly Lys Pro Gly Gln Arg Ser Ile Ala
               180                 185                 190
   Ala Ser Gly Gly Ile Leu Leu Trp Pro Gln Ser His Phe Asp Trp Val
           195                 200                 205
   Arg Pro Gly Ile Ile Leu Tyr Gly Val Ser Pro Leu Glu Asp Arg Ser
       210                 215                 220
   Thr Gly Ala Asp Phe Gly Cys Gln Pro Val Met Ser Leu Thr Ser Ser
   225                 230                 235                 240
   Leu Ile Ala Val Arg Glu His Lys Ala Gly Glu Pro Val Gly Tyr Gly
   245 250 255
   Gly Thr Trp Val Ser Glu Arg Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Pro Ile Val Gly Arg Val Ala Met Asp
   290 295 300
   Met Ile Cys Val Asp Leu Gly Pro Asn Ala Gln Asp Asn Ala Gly Asp
   305 310 315 320
   Pro Val Val Leu Trp Gly Glu Gly Leu Pro Val Glu Arg Ile Ala Glu
   325 330 335
   Met Thr Lys Val Ser Ala Tyr Glu Leu Ile Thr Arg Leu Thr Ser Arg
   340 345 350
   Val Ala Met Lys Tyr Ile Asp
   355
<210> 40
   <211> 358
   <212> PRT
   <213> Escherichia coli
<220>
   <221> misc_feature
   <222> (23)..(23)
   <223> Xaa can be any naturally occurring amino acid
<220>
   <221> misc_feature
   <222> (56)..(56)
   <223> Xaa can be any naturally occurring amino acid
<220>
   <221> misc_feature
   <222> (279)..(279)
   <223> Xaa can be any naturally occurring amino acid
<400> 40
   Met Gln Ala Ala Thr Val Val Ile Asn Arg Arg Ala Leu Arg His Asn
   1 5 10 15
   Leu Gln Arg Leu Arg Glu Xaa Ala Pro Ala Ser Lys Met Val Ala Val
   20 25 30
   Val Lys Ala Asn Ala Tyr Gly His Gly Leu Leu Glu Thr Ala Arg Thr
   35 40 45
   Leu Pro Asp Ala Asp Ala Phe Xaa Val Ala Arg Leu Glu Glu Ala Leu
       50                  55                  60
   Arg Leu Arg Ala Gly Gly Ile Thr Lys Pro Val Leu Leu Leu Glu Gly
   65                  70                  75                  80
   Phe Phe Asp Ala Arg Asp Leu Pro Thr Ile Ser Ala Gln His Phe His
                   85                  90                  95
   Thr Ala Val His Asn Glu Glu Gln Leu Ala Ala Leu Glu Glu Ala Ser
               100                 105                 110
   Leu Asp Glu Pro Val Thr Val Trp Met Lys Leu Asp Thr Gly Met His
           115                 120                 125
   Arg Leu Gly Val Arg Pro Glu Gln Ala Glu Ala Phe Tyr His Arg Leu
       130                 135                 140
   Thr Gln Cys Lys Asn Val Arg Gln Pro Val Asn Ile Val Ser His Phe
   145                 150                 155                 160
   Ala Arg Ala Asp Glu Pro Lys Cys Gly Ala Thr Glu Lys Gln Leu Ala
                   165                 170                 175
   Ile Phe Asn Thr Phe Cys Glu Gly Lys Pro Gly Gln Arg Ser Ile Ala
               180                 185                 190
   Ala Ser Gly Gly Ile Leu Leu Trp Pro Gln Ser His Phe Asp Trp Val
           195                 200                 205
   Arg Pro Gly Ile Ile Leu Tyr Gly Val Ser Pro Leu Glu Asp Arg Ser
       210                 215                 220
   Thr Gly Ala Asp Phe Gly Cys Gln Pro Val Met Ser Leu Thr Ser Ser
   225                 230                 235                 240
   Leu Ile Ala Val Arg Glu His Lys Ala Gly Glu Pro Val Gly Tyr Gly
                   245                 250                 255
   Gly Thr Trp Val Ser Glu Arg Asp Thr Arg Leu Gly Val Val Ala Met
               260                 265                 270
   Gly Tyr Gly Asp Gly Tyr Xaa Ala Ala Pro Ser Gly Thr Pro Val Leu
           275                 280                 285
   Val Asn Gly Arg Glu Val Pro Ile Val Gly Arg Val Ala Met Asp Met
       290                 295                 300
   Ile Cys Val Asp Leu Gly Pro Gln Ala Gln Asp Lys Ala Gly Asp Pro
   305                 310                 315                 320
   Val Ile Leu Trp Gly Glu Gly Leu Pro Val Glu Arg Ile Ala Glu Met
   325 330 335
   Thr Lys Val Ser Ala Tyr Glu Leu Ile Thr Arg Leu Thr Ser Arg Val
   340 345 350
   Ala Met Lys Tyr Val Asp
   355
<210> 41
   <211> 359
   <212> PRT
   <213> Artificial
<220>
   <223> synthetic gene
<400> 41
   Met Gln Ala Ala Thr Val Val Ile Asn Arg Arg Ala Leu Arg His Asn
   1 5 10 15
   Leu Gln Arg Leu Arg Glu Leu Ala Pro Ala Ser Lys Met Val Ala Val
   20 25 30
   Val Lys Ala Asn Ala Tyr Gly His Gly Leu Leu Glu Thr Ala Arg Thr
   35 40 45
   Leu Pro Asp Ala Asp Ala Phe Gly Val Ala Arg Leu Glu Glu Ala Leu
   50 55 60
   Arg Leu Arg Ala Gly Gly Ile Thr Lys Pro Val Leu Leu Leu Glu Gly
   65 70 75 80
   Phe Phe Asp Ala Arg Asp Leu Pro Thr Ile Ser Ala Gln His Phe His
   85 90 95
   Thr Ala Val His Asn Glu Gly Gln Leu Ala Ala Leu Glu Glu Ala Ser
   100 105 110
   Leu Asp Glu Pro Val Thr Val Trp Met Lys Leu Asp Thr Gly Met His
   115 120 125
   Arg Leu Gly Val Arg Pro Glu Gln Ala Glu Ala Phe Tyr His Arg Leu
   130 135 140
   Thr Gln Cys Lys Asn Val Arg Gln Pro Val Asn Ile Val Ser His Phe
   145 150 155 160
   Ala Arg Ala Asp Glu Pro Lys Cys Gly Ala Thr Glu Lys Gln Leu Ala
   165 170 175
   Ile Phe Asn Thr Phe Cys Glu Gly Lys Pro Gly Gln Arg Ser Ile Ala
   180 185 190
   Ala Ser Gly Gly Ile Leu Leu Trp Pro Gln Ser His Phe Asp Trp Val
           195                 200                 205
   Arg Pro Gly Ile Ile Leu Tyr Gly Val Ser Pro Leu Glu His Lys Pro
       210                 215                 220
   Trp Gly Pro Asp Phe Gly Phe Gln Pro Val Met Ser Leu Thr Ser Ser
   225                 230                 235                 240
   Leu Ile Ala Val Arg Asp His Lys Ala Gly Glu Pro Val Gly Tyr Gly
                   245                 250                 255
   Gly Thr Trp Val Ser Glu Arg Asp Thr Arg Leu Gly Val Val Ala Met
               260                 265                 270
   Gly Tyr Gly Asp Gly Tyr Pro Arg Ala Ala Pro Ser Gly Thr Pro Val
           275                 280                 285
   Leu Val Asn Gly Arg Glu Val Pro Ile Val Gly Arg Val Ala Met Asp
       290                 295                 300
   Met Ile Cys Val Asp Leu Gly Pro Gln Ala Gln Asp Lys Ala Gly Asp
   305                 310                 315                 320
   Pro Val Ile Leu Trp Gly Glu Gly Phe Pro Val Glu Arg Ile Ala Glu
                   325                 330                 335
   Met Thr Lys Val Ser Ala Tyr Glu Leu Ile Thr Arg Leu Thr Ser Arg
               340                 345                 350
   Val Ala Met Lys Tyr Val Asp
           355
<210> 42
   <211> 359
   <212> PRT
   <213> Escherichia coli CFT073
<400> 42
   Met Gln Ala Ala Thr Val Leu Ile Asn Arg Arg Ala Leu Arg His Asn
   1 5 10 15
   Leu Gln Arg Leu Arg Glu Leu Ala Pro Ala Ser Lys Leu Val Ala Val
   20 25 30
   Val Lys Ala Asn Ala Tyr Gly His Gly Leu Ile Glu Thr Ala Arg Thr
   35 40 45
   Leu Pro Asp Ala Asp Ala Phe Gly Val Ala Arg Leu Glu Glu Ala Leu
   50 55 60
   Arg Leu Arg Ala Gly Gly Ile Thr Arg Pro Ile Leu Leu Leu Glu Gly
   65 70 75 80
   Phe Phe Glu Ala Asp Asp Leu Pro Thr Ile Ser Ala Glu His Leu His
   85 90 95
   Thr Ala Val His Asn Glu Glu Gln Leu Val Ala Leu Glu Asn Ala Glu
   100 105 110
   Leu Lys Glu Pro Val Thr Val Trp Met Lys Leu Asp Thr Gly Met His
   115 120 125
   Arg Leu Gly Val Leu Pro Glu Gln Ala Glu Ala Phe Tyr Gln Arg Leu
   130 135 140
   Ser Gln Cys Lys Asn Val Arg Gln Pro Val Asn Ile Val Ser His Phe
   145 150 155 160
   Ala Arg Ala Asp Glu Pro Gln Ser Gly Ala Thr Glu Lys Gln Leu Asp
   165 170 175
   Ile Phe Asn Thr Phe Cys Glu Gly Lys Pro Gly Gln Arg Ser Ile Ala
   180 185 190
   Ala Ser Gly Gly Ile Leu Leu Trp Pro Gln Ser His Phe Asp Trp Ala
   195 200 205
   Arg Pro Gly Ile Ile Leu Tyr Gly Val Ser Pro Leu Glu Asp Gly Thr
   210 215 220
   Thr Gly Ala Asp Phe Gly Cys Gln Pro Val Met Ser Leu Thr Ser Ser
   225 230 235 240
   Leu Ile Ala Val Arg Glu His Lys Ala Gly Glu Pro Val Gly Tyr Gly
   245 250 255
   Gly Thr Trp Val Ser Glu Arg Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Pro Ile Val Gly Arg Val Ala Met Asp
   290 295 300
   Met Ile Cys Val Asp Leu Gly Pro Gln Ala Gln Asp Lys Ala Gly Asp
   305 310 315 320
   Pro Val Ile Leu Trp Gly Glu Gly Leu Pro Val Glu Arg Ile Ala Glu
   325 330 335
   Met Thr Lys Val Ser Ala Tyr Glu Leu Ile Thr Arg Leu Thr Ser Arg
   340 345 350
   Val Ala Met Lys Tyr Val Asp
   355
<210> 43
   <211> 359
   <212> PRT
   <213> Salmonella enterica subsp. enterica serovar
<400> 43
   Met Gln Ala Ala Thr Val Val Ile Asn Arg Arg Ala Leu Arg His Asn
   1 5 10 15
   Leu Gln Arg Leu Arg Glu Leu Ala Pro Ala Ser Lys Leu Val Ala Val
   20 25 30
   Val Lys Ala Asn Ala Tyr Gly His Gly Leu Leu Glu Thr Ala Arg Thr
   35 40 45
   Leu Pro Asp Ala Asp Ala Phe Gly Val Ala Arg Leu Glu Glu Ala Leu
   50 55 60
   Arg Leu Arg Ala Gly Gly Ile Thr Gln Pro Ile Leu Leu Leu Glu Gly
   65 70 75 80
   Phe Phe Asp Ala Ala Asp Leu Pro Thr Ile Ser Ala Gln Cys Leu His
   85 90 95
   Thr Ala Val His Asn Gln Glu Gln Leu Ala Ala Leu Glu Ala Val Glu
   100 105 110
   Leu Ala Glu Pro Val Thr Val Trp Met Lys Leu Asp Thr Gly Met His
   115 120 125
   Arg Leu Gly Val Arg Pro Glu Glu Ala Glu Ala Phe Tyr Gln Arg Leu
   130 135 140
   Thr His Cys Lys Asn Val Arg Gln Pro Val Asn Ile Val Ser His Phe
   145 150 155 160
   Ala Arg Ala Asp Glu Pro Glu Cys Gly Ala Thr Glu His Gln Leu Asp
   165 170 175
   Ile Phe Ser Ala Phe Cys Gln Gly Lys Pro Gly Gln Arg Ser Ile Ala
   180 185 190
   Ala Ser Gly Gly Ile Leu Leu Trp Pro Gln Ser His Phe Asp Trp Ala
   195 200 205
   Arg Pro Gly Ile Ile Leu Tyr Gly Val Ser Pro Leu Glu His Lys Pro
   210 215 220
   Trp Gly Pro Asp Phe Gly Phe Gln Pro Val Met Ser Leu Thr Ser Ser
   225 230 235 240
   Leu Ile Ala Val Arg Asp His Lys Ala Gly Glu Pro Val Gly Tyr Gly
   245 250 255
   Gly Thr Trp Val Ser Glu Arg Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Pro Ile Val Gly Arg Val Ala Met Asp
   290 295 300
   Met Ile Cys Val Asp Leu Gly Pro Asn Ala Gln Asp Asn Ala Gly Asp
   305 310 315 320
   Pro Val Val Leu Trp Gly Glu Gly Leu Pro Val Glu Arg Ile Ala Glu
   325 330 335
   Met Thr Lys Val Ser Ala Tyr Glu Leu Ile Thr Arg Leu Thr Ser Arg
   340 345 350
   Val Ala Met Lys Tyr Ile Asp
   355
<210> 44
   <211> 359
   <212> PRT
   <213> Enterobacter sp. 638
<400> 44
   Met Gln Ala Ala Thr Val Val Ile Asn Arg Arg Ala Leu Arg His Asn
   1 5 10 15
   Leu Gln Arg Leu Arg Glu Leu Ala Pro Ala Ser Lys Leu Val Ala Val
   20 25 30
   Val Lys Ala Asn Ala Tyr Gly His Gly Leu Leu Glu Thr Ala Arg Thr
   35 40 45
   Leu Pro Asp Ala Asp Ala Phe Gly Val Ala Arg Leu Glu Glu Ala Leu
   50 55 60
   Arg Leu Arg Ala Gly Gly Ile Thr Gln Pro Ile Leu Leu Leu Glu Gly
   65 70 75 80
   Phe Phe Glu Ala Thr Asp Leu Thr Thr Ile Ala Asp Gln His Leu His
   85 90 95
   Thr Ala Ile His Asn Glu Glu Gln Leu Leu Ala Leu Glu Thr Ala Glu
   100 105 110
   Leu Ser Glu Pro Val Thr Val Trp Met Lys Leu Asp Thr Gly Met His
   115 120 125
   Arg Leu Gly Val Arg Pro Glu Gln Ala Glu Ala Phe Tyr Gln Arg Leu
   130 135 140
   Ser Gln Cys Lys Asn Val His Gln Pro Val Asn Ile Val Ser His Phe
   145 150 155 160
   Ala Arg Ala Asp Glu Pro Glu Cys Gly Ala Thr Glu Gln Gln Leu Asp
   165 170 175
   Ile Phe Asn Thr Phe Cys Glu Gly Lys Pro Gly Met Arg Ser Ile Ala
   180 185 190
   Ala Ser Gly Gly Ile Leu Leu Trp Pro Gln Ser His Phe Asp Trp Ala
   195 200 205
   Arg Pro Gly Ile Ile Leu Tyr Gly Val Ser Pro Leu Glu Asn Lys Pro
   210 215 220
   Trp Gly Pro Asp Phe Gly Leu Gln Pro Val Met Ser Leu Val Ser Asn
   225 230 235 240
   Leu Ile Ala Val Arg Glu His Lys Ala Gly Glu Pro Val Gly Tyr Gly
   245 250 255
   Gly Thr Trp Val Ser Glu Ser Asp Thr Arg Leu Gly Val Val Ala Met
   260 265 270
   Gly Tyr Gly Asp Gly Tyr Pro Arg Ala Ala Pro Ser Gly Thr Pro Val
   275 280 285
   Leu Val Asn Gly Arg Glu Val Lys Ile Val Gly Arg Val Ala Met Asp
   290 295 300
   Met Ile Cys Val Asp Leu Gly Pro Asp Ala Gln Asp Lys Ala Gly Asp
   305 310 315 320
   Ala Val Val Leu Trp Gly Glu Gly Leu Pro Val Glu Arg Ile Ala Glu
   325 330 335
   Ile Thr Lys Val Ser Ala Tyr Glu Leu Ile Thr Arg Leu Thr Ser Arg
   340 345 350
   Val Ala Met Lys Tyr Ile Asp
   355

## Description

### FIELD OF THE INVENTION

The invention relates to the control of nematodes, in particular the control of soybean cyst nematodes. Disclosed herein are methods of producing transgenic plants with increased nematode resistance, expression vectors comprising polynucleotides encoding for functional proteins, and transgenic plants and seeds generated thereof.

### BACKGROUND OF THE INVENTION

Nematodes are microscopic wormlike animals that feed on the roots, leaves, and stems of more than 2,000 vegetables, fruits, and ornamental plants, causing an estimated $100 billion crop loss worldwide. One common type of nematode is the root-knot nematode (RKN), whose feeding causes the characteristic galls on roots on a wide variety of plant species. Other root-feeding nematodes are the cyst- and lesion-types, which are more host specific.

Nematodes are present throughout the United States, but are mostly a problem in warm, humid areas of the South and West, and in sandy soils. Soybean cyst nematode (SCN), Heterodera glycines, was first discovered in the United States in North Carolina in 1954. It is the most serious pest of soybean plants. Some areas are so heavily infested by SCN that soybean production is no longer economically possible without control measures. Although soybean is the major economic crop attacked by SCN, SCN parasitizes some fifty hosts in total, including field crops, vegetables, ornamentals, and weeds.

Signs of nematode damage include stunting and yellowing of leaves, and wilting of the plants during hot periods. However, nematodes, including SCN, can cause significant yield loss without obvious above-ground symptoms. In addition, roots infected with SCN are dwarfed or stunted. Nematode infestation can decrease the number of nitrogen-fixing nodules on the roots, and may make the roots more susceptible to attacks by other soil-borne plant pathogens.

The nematode life cycle has three major stages: egg, juvenile, and adult. The life cycle varies between species of nematodes. For example, the SCN life cycle can usually be completed in 24 to 30 days under optimum conditions whereas other species can take as long as a year, or longer, to complete the life cycle. When temperature and moisture levels become adequate in the spring, worm-shaped juveniles hatch from eggs in the soil. These juveniles are the only life stage of the nematode that can infect soybean roots.
The life cycle of SCN has been the subject of many studies and therefore can be used as an example for understanding a nematode life cycle. After penetrating the soybean roots, SCN juveniles move through the root until they contact vascular tissue, where they stop and start to feed. The nematode injects secretions that modify certain root cells and transform them into specialized feeding sites. The root cells are morphologically transformed into large multinucleate syncytia (or giant cells in the case of RKN), which are used as a source of nutrients for the nematodes. The actively feeding nematodes thus steal essential nutrients from the plant resulting in yield loss. As the nematodes feed, they swell and eventually female nematodes become so large that they break through the root tissue and are exposed on the surface of the root.

Male SCN nematodes; which are not swollen as adults, migrate out of the root into the soil and fertilize the lemon-shaped adult females. The males then die, while the females remain attached to the root system and continue to feed. The eggs in the swollen females begin developing, initially in a mass or egg sac outside the body, then later within the body cavity. Eventually the entire body cavity of the adult female is filled with eggs, and the female nematode dies. It is the egg-filled body of the dead female that is referred to as the cyst. Cysts eventually dislodge and are found free in the soil. The walls of the cyst become very tough, providing excellent protection for the approximately 200 to 400 eggs contained within. SCN eggs survive within the cyst until proper hatching conditions occur. Although many of the eggs may hatch within the first year, many also will survive within the cysts for several years.

Nematodes can move through the soil only a few inches per year on its own power. However, nematode infestation can be spread substantial distances in a variety of ways. Anything that can move infested soil is capable of spreading the infestation, including farm machinery, vehicles and tools, wind, water, animals, and farm workers. Seed sized particles of soil often contaminate harvested seed. Consequently, nematode infestation can be spread when contaminated seed from infested fields is planted in non-infested fields. There is even evidence that certain nematode species can be spread by birds. Only some of these causes can be prevented.

Traditional practices for managing nematode infestation include: maintaining proper soil nutrients and soil pH levels in nematode-infested land; controlling other plant diseases, as well as insect and weed pests; using sanitation practices such as plowing, planting, and cultivating of nematode-infested fields only after working non-infested fields; cleaning equipment thoroughly with high pressure water or steam after working in infested fields; not using seed grown on infested land for planting non-infested fields unless the seed has been property cleaned; rotating infested fields and alternating host crops with non-host crops; using nematicides; and planting resistant plant varieties.

Methods have been proposed for the genetic transformation of plants in order to confer increased resistance to plant parasitic nematodes. U.S. Patent Nos. 5,589,622 and 5,824,876 are directed to the identification of plant genes expressed specifically in or adjacent to the feeding site of the plant after attachment by the nematode.

Alanine racemase (EC 5.1.1.1) catalyzes the interconversion of alanine L- and D-enantiomers and represents the first committed step involved in bacterial cell wall biosynthesis. D-alanine is an essential component of cell wall peptidoglycan (mureine) in all bacteria and is produced by the racemation of L-alanine. Activity of alanine racemase in E. coli is due to two distinct gene products. One alanine racemase (Alr) is constitutive/ low abundance and is encoded by alr (Neidhardt et al., J. Biol. Chem. 1989, 15:264(5):2393-6). The other alanine racemase, DadX, is induced by D- or L-alanine and repressed by glucose and is encoded by the DadX gene (Hennig et al., Mol Gen Genet 1985, 198(2):315-22).

Notwithstanding the foregoing, there is a need to identify safe and effective compositions and methods for controlling plant parasitic nematodes, and for the production of plants having increased resistance to plant parasitic nematodes.

### SUMMARY OF THE INVENTION

The present inventiors found that expressing a transgene comprising an alanine racemase gene in a plant can confer nematode resistance to the plant. The present invention provides transgenic plants and seeds, and methods to overcome, or at least alleviate, nematode infestation in crop plants.

Therefore, in one embodiment, the invention concerns a transgenic plant comprising an expression vector comprising an isolated polynucleotide encoding an alanine racemase. Preferably, the alanine racemase encoding polynucleotide is over-expressed in nematode-induced syncytia.

Another embodiment of the invention provides a transgenic seed that is true breeding for an alanine racemase transgene.

Another embodiment of the invention relates to the use of an expression vector comprising a transcription regulatory element operably linked to a polynucleotide that encodes an alanine racemase, wherein expression of the polynucleotide is confers nematode resistance to a transgenic plant. Preferably, the expression vector also comprises a promoter operably linked to the alanine racemase encoding polynucleotide, the promoter being capable of directing expression of the alanine racemase encoding polynucleotide in roots or in syncytia of plants infected with nematodes.

In another embodiment, the invention provides a method of producing a transgenic plant having increased nematode resistance, wherein the method comprises the steps of introducing into the plant an expression vector comprising a promoter operably linked to an alanine racemase encoding polynucleotide, wherein expression of the polynucleotide confers increased nematode resistance to the plant.

### BRIEF DECRIPTION OF THE DRAWINGS

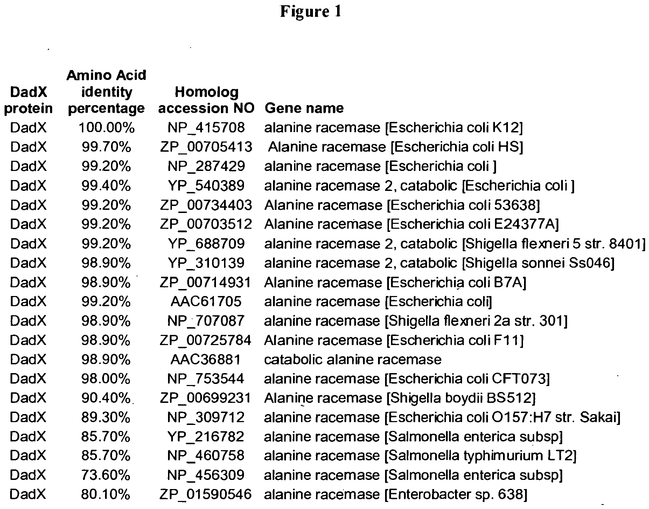

Figure 1 shows amino acid identity percentage of DadX homologs to DadX protein (SEQ ID NO:6).

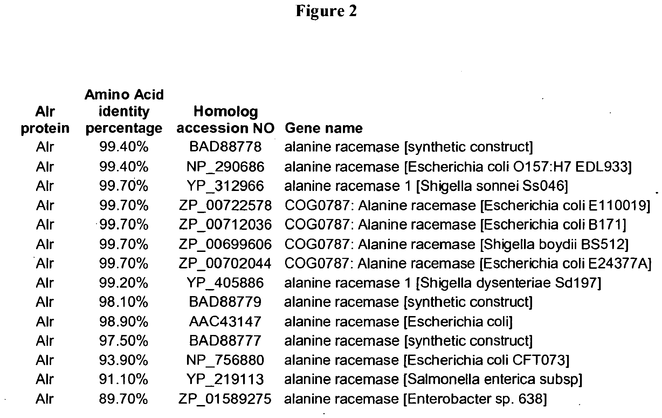

Figure 2 shows the amino acid identity percentage of Alr homologs to Alr protein (SEQ ID NO:8).

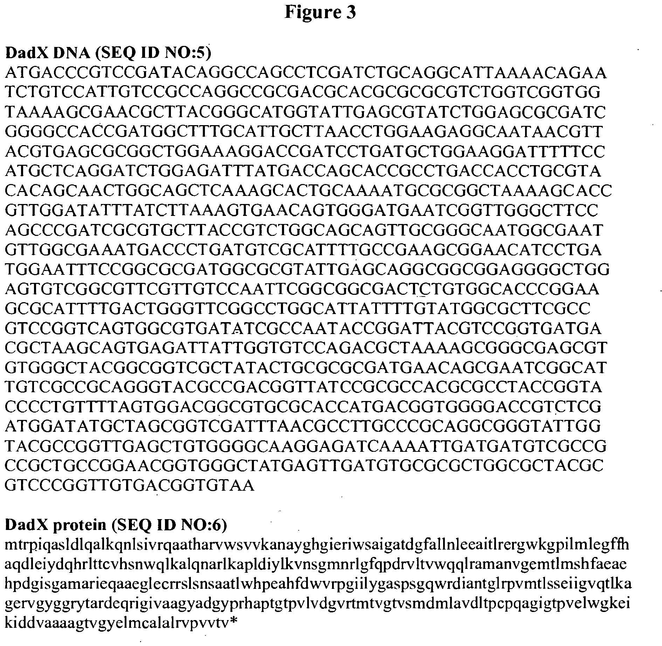

Figure 3 shows the DNA and protein sequences of DadX gene.

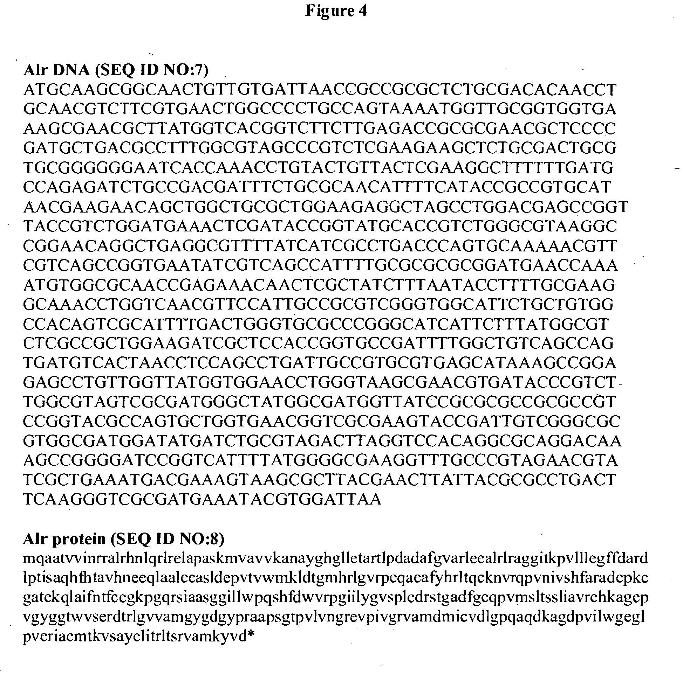

Figure 4 shows the DNA and protein sequences of Alr gene.

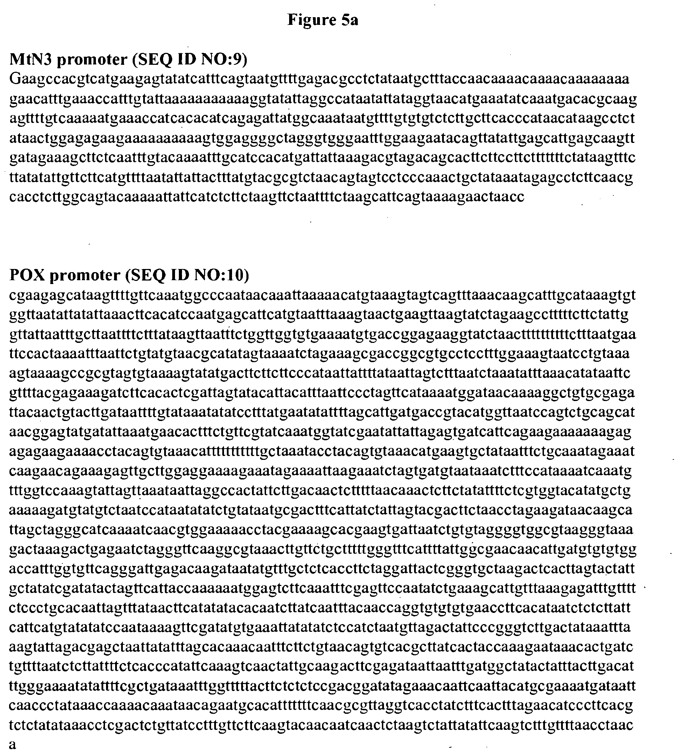

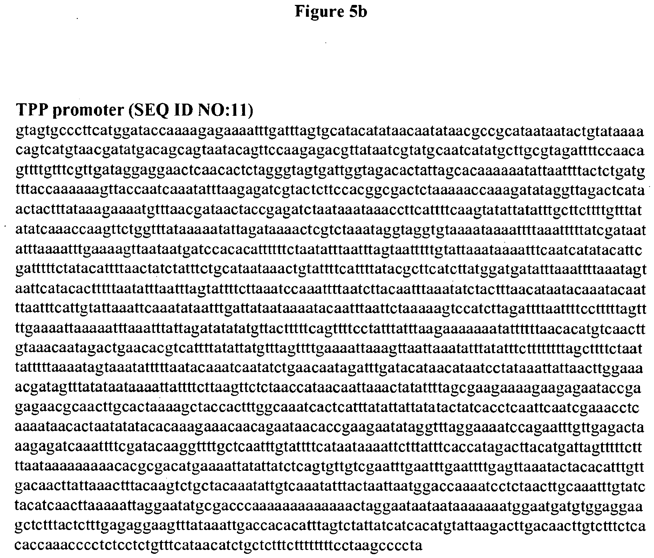

Figure 5a and 5b show the DNA sequences for MTN3, POX and TPP promoters.

### DETAILED DESCRIPTION OF THE PREFERRED EMBODIMENTS

The present invention may be understood more readily by reference to the following detailed description of the embodiments of the invention and the examples included herein. Unless otherwise noted, the terms used herein are to be understood according to conventional usage by those of ordinary skill in the relevant art.

Throughout this application, various patent and scientific publications are referenced. The disclosures of all of these publications and those references cited within those publications in their entireties are hereby incorporated by reference into this application in order to more fully describe the state of the art to which this invention pertains. Abbreviations and nomenclature, where employed, are deemed standard in the field and commonly used in professional journals such as those cited herein. As used herein and in the appended claims, the singular form "a", "an", or "the" includes plural reference unless the context clearly dictates otherwise. As used herein, the word "or" means any one member of a particular list and also includes any combination of members of that list.

The term "about" is used herein to mean approximately, roughly, around, or in the regions of. When the term "about" is used in conjunction with a numerical range, it modifies that range by extending the boundaries above and below the numerical values set forth. In general, the term "about" is used herein to modify a numerical value above and below the stated value by a variance of 10 percent, up or down (higher or lower).
As used herein, the word "nucleic acid", "nucleotide", or "polynucleotide" is intended to include DNA molecules (e.g., cDNA or genomic DNA), RNA molecules (e.g., mRNA), natural occurring, mutated, synthetic DNA or RNA molecules, and analogs of the DNA or RNA generated using nucleotide analogs. It can be single-stranded or double-stranded. Such nucleic acids or polynucleotides include, but are not limited to, coding sequences of structural genes, anti-sense sequences, and non-coding regulatory sequences that do not encode mRNAs or protein products. A polynucleotide may encode for an agronomically valuable or a phenotypic trait.

As used herein, an "isolated" polynucleotide is substantially free of other cellular materials or culture medium when produced by recombinant techniques, or substantially free of chemical precursors when chemically synthesized.

The term "gene" is used broadly to refer to any segment of nucleic acid associated with a biological function. Thus, genes include introns and exons as in genomic sequence, or just the coding sequences as in cDNAs and/or the regulatory sequences required for their expression. For example, gene refers to a nucleic acid fragment that expresses mRNA or functional RNA, or encodes a specific protein, and which includes regulatory sequences.

The terms "polypeptide" and "protein" are used interchangeably herein to refer to a polymer of consecutive amino acid residues.

The term "operably linked" or "functionally linked" as used herein refers to the association of nucleic acid sequences on single nucleic acid fragment so that the function of one is affected by the other. For example, a regulatory DNA is said to be "operably linked to" a DNA that expresses an RNA or encodes a polypeptide if the two DNAs are situated such that the regulatory DNA affects the expression of the coding DNA.

The term "specific expression" as used herein refers to the expression of gene products that is limited to one or a few plant tissues (special limitation) and/or to one or a few plant developmental stages (temporal limitation). It is acknowledged that hardly a true specificity exists: promoters seem to be preferably switched on in some tissues, while in other tissues there can be no or only little activity. This phenomenon is known as leaky expression. However, with specific expression as defined herein expression in one or a few plant tissues or specific sites in a plant.

The term "promoter" as used herein refers to a DNA sequence which, when ligated to a nucleotide sequence of interest, is capable of controlling the transcription of the nucleotide sequence of interest into mRNA. A promoter is typically, though not necessarily, located 5' (e.g., upstream) of a nucleotide of interest (e.g., proximal to the transcriptional start site of a structural gene) whose transcription into mRNA it controls, and provides a site for specific binding by RNA polymerase and other transcription factors for initiation of transcription.

The term "transcription regulatory element" as used herein refers to a polynucleotide that is capable of regulating the transcription of an operably linked polynucleotide. It includes, but not limited to, promoters, enhancers, introns, 5' UTRs, and 3' UTRs.

As used herein, the term "vector" refers to a nucleic acid molecule capable of transporting another nucleic acid to which it has been linked. One type of vector is a "plasmid", which refers to a circular double stranded DNA loop into which additional DNA segments can be ligated. In the present specification, "plasmid" and "vector" can be used interchangeably as the plasmid is the most commonly used form of vector. A vector can be a binary vector or a T-DNA that comprises the left border and the right border and may include a gene of interest in between. The term "expression vector" as used herein means a vector capable of directing expression of a particular nucleotide in an appropriate host cell. An expression vector comprises a regulatory nucleic acid element operably linked to a nucleic acid of interest, which is - optionally - operably linked to a termination signal and/or other regulatory elements.

The term "homologs" as used herein refers to a gene related to a second gene by descent from a common ancestral DNA sequence. The term "homologs" may apply to the relationship between genes separated by the event of speciation (e.g., orthologs) or to the relationship between genes separated by the event of genetic duplication (e.g., paralogs).

As used herein, the term "orthologs" refers to genes from different species, but that have evolved from a common ancestral gene by speciation. Orthologs retain the same function in the course of evolution. Orthologs encode proteins having the same or similar functions. As used herein, the term "paralogs" refers to genes that are related by duplication within a genome. Paralogs usually have different functions or new functions, but these functions may be related.

As used herein, the term "hybridizes under stringent conditions" is intended to describe conditions for hybridization and washing under which nucleotide sequences at least 60% similar or identical to each other typically remain hybridized to each other. In another embodiment, the conditions are such that sequences at least about 65%, or at least about 70%, or at least about 75% or more similar or identical to each other typically remain hybridized to each other. Such stringent conditions are known to those skilled in the art and described as below. A preferred, non-limiting example of stringent conditions are hybridization in 6X sodium chloride/sodium citrate (SSC) at about 45°C, followed by one or more washes in 0.2X SSC, 0.1% SDS at 50-65°C.

The term "sequence identity" or "identity" in the context of two nucleic acid or polypeptide sequences makes reference to those positions in the two sequences where identical pairs of symbols fall together when the sequences are aligned for maximum correspondence over a specified comparison window, for example, either the entire sequence as in a global alignment or the region of similarity in a local alignment. When percentage of sequence identity is used in reference to proteins it is recognized that residue positions that are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity". Means for making this adjustment are well known to those of skilled in the art. Typically this involves scoring a conservative substitution as a partial match rather than a mismatch, thereby increasing the percentage of sequence similarity.

As used herein, "percentage of sequence identity" or "sequence identity percentage" denotes a value determined by first noting in two optimally aligned sequences over a comparison window, either globally or locally, at each constituent position as to whether the identical nucleic acid base or amino acid residue occurs in both sequences, denoted a match, or does not, denoted a mismatch. As said alignment are constructed by optimizing the number of matching bases, while concurrently allowing both for mismatches at any position and for the introduction of arbitrarily-sized gaps, or null or empty regions where to do so increases the significance or quality of the alignment, the calculation determines the total number of positions for which the match condition exists, and then divides this number by the total number of positions in the window of comparison, and lastly multiplies the result by 100 to yield the percentage of sequence identity. "Percentage of sequence similarity" for protein sequences can be calculated using the same principle, wherein the conservative substitution is calculated as a partial rather than a complete mismatch. Thus, for example, where an identical amino acid is given a score of 1 and a non-conservative substitution is given a score of zero, a conservative substitution is given a score between zero and 1. The scoring of conservative substitutions can be obtained from amino acid matrices known in the art, for example, Blosum or PAM matrices.

Methods of alignment of sequences for comparison are well known in the art. The determination of percent identity or percent similarity (for proteins) between two sequences can be accomplished using a mathematical algorithm. Preferred, non-limiting examples of such mathematical algorithms are, the algorithm of Myers and Miller (Bioinformatics, 4(1):11-17, 1988), the Needleman-Wunsch global alignment (J Mol Biol. 48(3):443-53, 1970), the Smith-Waterman local alignment (J. Mol. Biol., 147:195-197, 1981), the search-for-similarity-method of Pearson and Lipman (PNAS, 85(8): 2444-2448, 1988), the algorithm of Karlin and Altschul (J. Mol. Biol., 215(3):403-410, 1990; PNAS, 90:5873-5877,1993). Computer implementations of these mathematical algorithms can be utilized for comparison of sequences to determine sequence identity or to identify homologs. Such implementations include, but are not limited to, the programs described below.

The term "conserved region" or "conserved domain" as used herein refers to a region in heterologous polynucleotide or polypeptide sequences where there is a relatively high degree of sequence identity between the distinct sequences. The "conserved region" can be identified, for example, from the multiple sequence alignment using the Clustal W algorithm.

The term "cell" or "plant cell" as used herein refers to single cell, and also includes a population of cells. The population may be a pure population comprising one cell type. Likewise, the population may comprise more than one cell type. A plant cell within the meaning of the invention may be isolated (e.g., in suspension culture) or comprised in a plant tissue, plant organ or plant at any developmental stage.

The term "tissue" with respect to a plant (or "plant tissue") means arrangement of multiple plant cells, including differentiated and undifferentiated tissues of plants. Plant tissues may constitute part of a plant organ (e.g., the epidermis of a plant leaf) but may also constitute tumor tissues (e.g., callus tissue) and various types of cells in culture (e.g., single cells, protoplasts, embryos, calli, protocorm-like bodies, etc.). Plant tissues may be in planta, in organ culture, tissue culture, or cell culture.

The term "organ" with respect to a plant (or "plant organ") means parts of a plant and may include, but not limited to, for example roots, fruits, shoots, stems, leaves, hypocotyls, cotyledons, anthers, sepals, petals, pollen, seeds, etc.

The term "plant" as used herein can, depending on context, be understood to refer to whole plants, plant cells, plant organs, plant seeds, and progeny of same. The word "plant" also refers to any plant, particularly, to seed plant, and may include, but not limited to, crop plants. Plant parts include, but are not limited to, stems, roots, shoots, fruits, ovules, stamens, leaves, embryos, meristematic regions, callus tissue, gametophytes, sporophytes, pollen, microspores, hypocotyls, cotyledons, anthers, sepals, petals, pollen, seeds and the like. The class of plants that can be used in the method of the invention is generally as broad as the class of higher and lower plants amenable to transformation techniques, including angiosperms (monocotyledonous and dicotyledonous plants), gymnosperms, ferns, horsetails, psilophytes, bryophytes, and multicellular algae...

The term "transgenic" as used herein is intended to refer to cells and/or plants which contain a transgene, or whose genome has been altered by the introduction of a transgene, or that have incorporated exogenous genes or polynucleotides. Transgenic cells, tissues, organs and plants may be produced by several methods including the introduction of a "transgene" comprising polynucleotide (usually DNA) into a target cell or integration of the transgene into a chromosome of a target cell by way of human intervention, such as by the methods described herein.

The term "true breeding" as used herein refers to a variety of plant for a particular trait if it is genetically homozygous for that trait to the extent that, when the true-breeding variety is self-pollinated, a significant amount of independent segregation of the trait among the progeny is not observed.

The term "wild type" as used herein refers to a plant cell, seed, plant component, plant tissue, plant organ, or whole plant that has not been genetically modified or treated in an experimental sense.

The term "control plant" or "wild type plant" as used herein refers to a plant cell, an explant, seed, plant component, plant tissue, plant organ, or whole plant used to compare against transgenic or genetically modified plant for the purpose of identifying an enhanced phenotype or a desirable trait in the transgenic or genetically modified plant. A "control plant" may in some cases be a transgenic plant line that comprises an empty vector or marker gene, but does not contain the recombinant polynucleotide of interest that is present in the transgenic or genetically modified plant being evaluated. A control plant may be a plant of the same line or variety as the transgenic or genetically modified plant being tested, or it may be another line or variety, such as a plant known to have a specific phenotype, characteristic, or known genotype. A suitable control plant would include a genetically unaltered or non-transgenic plant of the parental line used to generate a transgenic plant herein.

The term "resistant to nematode infection" or "a plant having nematode resistance" as used herein refers to the ability of a plant to avoid infection by nematodes, to kill nematodes or to hamper, reduce or stop the development, growth or multiplication of nematodes. This might be archieved by an active process, e.g. by producing a substance detrimental to the nematode, or by a passive process, like having a reduced nutritional value for the nematode or not developing structures induced by the nematode feeding site like syncytial or giant cells. The level of nematode resistance of a plant can be determined in various ways, e.g. by counting the nematodes being able to establish parasitism on that plant, or measuring development times of nematodes, proportion of male and female nematodes or the number of cysts or nematode eggs produced. A plant with increased resistance to nematode infection is a plant, which is more resistant to nematode infection in comparison to another plant having a similar or preferably a identical genotype while lacking the gene or genes conferring increased resistance to nematodes, e.g., a control or wild type plant.

The term "feeding site" or "syncytia site" are used interchangeably and refer as used herein to the feeding site formed in plant roots after nematode infestation. The site is used as a source of nutrients for the nematodes. Syncytia is the feeding site for cyst nematodes and giant cells are the feeding sites of root knot nematodes.

In one embodiment, the invention provides a transgenic plant transformed with an expression vector comprising an isolated alanine racemase encoding polynucleotide, wherein expression of the polynucleotide confers increased nematode resistance to the plant. Preferably, the alanine racemase encoding polynucleotide is selected from the group consisting of a polynucleotide having a sequence as defined in SEQ ID NO:5 or 7; a polynucleotide encoding a polypeptide having a sequence as defined in SEQ ID NO:6 or 8; a polynucleotide having at least 70% sequence identity to a polynucleotide having a sequence as defined in SEQ ID NO:5 or 7; a polynucleotide encoding a polypeptide having at least 70% sequence identity to a polypeptide having a sequence as defined in SEQ ID NO:6 or 8; a polynucleotide that hybridizes under stringent conditions to a polynucleotide having a sequence as defined in SEQ ID NO:5 or 7; a polynucleotide that hybridizes under stringent conditions to a polynucleotide encoding a polypeptide having a sequence as defined in SEQ ID NO:6 or 8; a polynucleotide encoding a polypeptide having a sequence as defined in any of SEQ ID NOs: 12 through 44, and a polynucleotide encoding a polypeptide having at least 90% sequence identity to any of the sequences as defined in any of SEQ ID NOs: 12 through 44.

An alanine racemase encoding polynucleotide as defined herein also encompasses homologs, orthologs, paralogs, and allelic variants of the alanine racemase encoding polynucleotide of SEQ ID NO:5 or 7, or a polynucleotide encoding a polypeptide having a sequence as defined in SEQ ID NO:6 or 8 or as defined in any of SEQ ID NOs: 12 through 44. As used herein, the term "allelic variant" refers to a polynucleotide containing polymorphisms that lead to changes in the amino acid sequences of a protein encoded by the nucleotide and that exist within a natural population (e.g., a plant species or variety). Such natural allelic variations can typically result in 1-5% variance in a polynucleotide encoding a protein, or 1-5% variance in the encoded protein. Allelic variants can be identified by sequencing the nucleic acid of interest in a number of different plants, which can be readily carried out by using, for example, hybridization probes to identify the same gene genetic locus in those plants. Any and all such nucleic acid variations in a polynucleotide and resulting amino acid polymorphisms or variations of a protein that are the result of natural allelic variation and that do not alter the functional activity of the encoded protein, are intended to be within the scope of the invention. To clone allelic variants or homologs of the polynucleotides of the invention, the sequence information given herein can be used. For example the primers described by SEQ ID NO: 1, 2, 3 and 4 can be used to clone allelic variants or homologs.

In yet another embodiment, the plant may be a plant selected from the group consisting of monocotyledonous plants and dicotyledonous plants. The plant can be from a genus selected from the group consisting of maize, wheat, rice, barley, oat, rye, sorghum, banana, and ryegrass. The plant can be from a genus selected from the group consisting of pea, alfalfa, soybean, carrot, celery, tomato, potato, cotton, tobacco, pepper, oilseed rape, beet, cabbage, cauliflower, broccoli, lettuce and Arabidopsis thaliana.

The present invention also provides transgenic seed that is true-breeding for an alanine racemase encoding polynucleotide, and parts from transgenic plants that comprise the alanine racemase encoding polynucleotide, and progeny plants from such a plant, including hybrids and inbreds. The invention also provides a method of plant breeding, e.g., to prepare a crossed fertile transgenic plant. The method comprises crossing a fertile transgenic plant comprising a particular expression vector of the invention with itself or with a second plant, e.g., one lacking the particular expression vector, to prepare the seed of a crossed fertile transgenic plant comprising the particular expression vector. The seed is then planted to obtain a crossed fertile transgenic plant. The plant may be a monocot or a dicot. The crossed fertile transgenic plant may have the particular expression vector inherited through a female parent or through a male parent. The second plant may be an inbred plant. The crossed fertile transgenic may be a hybrid. Also included within the present invention are seeds of any of these crossed fertile transgenic plants.

Another embodiment of the invention relates to the use of an expression vector comprising a transcription regulatory element operably linked to an alanine racemase encoding polynucleotide, wherein expression of the polynucleotide confers increased nematode resistance to a transgenic plant. In one embodiment, the transcription regulatory element is a promoter capable of regulating constitutive expression of an operably linked polynucleotide. A "constitutive promoter" refers to a promoter that is able to express the open reading frame or the regulatory element that it controls in all or nearly all of the plant tissues during all or nearly all developmental stages of the plant. Constitutive promoters include, but not limited to, the 35S CaMV promoter from plant viruses (Franck et al., Cell 21:285-294, 1980), the Nos promoter (An G. at al., The Plant Cell 3:225-233, 1990), the ubiquitin promoter (Christensen et al., Plant Mol. Biol. 12:619-632, 1992 and 18:581-8, 1991), the MAS promoter (Velten et al., EMBO J. 3:2723-30, 1984), the maize H3 histone promoter (Lepetit et al., Mol Gen. Genet 231:276-85, 1992), the ALS promoter (WO96/30530), the 19S CaMV promoter (US 5,352,605), the super-promoter (US 5,955,646), the figwort mosaic virus promoter (US 6,051,753), the rice actin promoter (US 5,641,876), and the Rubisco small subunit promoter (US 4,962,028).

In another embodiment, the transcription regulatory element is a regulated promoter. A "regulated promoter" refers to a promoter that directs gene expression not constitutively, but in a temporally and/or spatially manner, and includes both tissue-specific and inducible promoters. Different promoters may direct the expression of a gene or regulatory element in different tissues or cell types, or at different stages of development, or in response to different environmental conditions.

A "tissue-specific promoter" refers to a regulated promoter that is not expressed in all plant cells but only in one or more cell types in specific organs (such as leaves or seeds), specific tissues (such as embryo or cotyledon), or specific cell types (such as leaf parenchyma or seed storage cells). These also include promoters that are temporally regulated, such as in early or late embryogenesis, during fruit ripening in developing seeds or fruit, in fully differentiated leaf, or at the onset of sequence. Suitable promoters include the napin-gene promoter from rapeseed (US 5,608,152), the USP-promoter from Vicia faba (Baeumlein et al., Mol Gen Genet. 225(3):459-67, 1991), the oleosin-promoter from Arabidopsis (WO 98/45461), the phaseolin-promoter from Phaseolus vulgaris (US 5,504,200), the Bce4-promoter from Brassica (WO 91/13980) or the legumin B4 promoter (LeB4; Baeumlein et al., Plant Journal, 2(2):233-9, 1992) as well as promoters conferring seed specific expression in monocot plants like maize, barley, wheat, rye, rice, etc. Suitable promoters to note are the Ipt2 or Ipt1-gene promoter from barley (WO 95/15389 and WO 95/23230) or those described in WO 99/16890 (promoters from the barley hordein-gene, rice glutelin gene, rice oryzin gene, rice prolamin gene, wheat gliadin gene, wheat glutelin gene, maize zein gene, oat glutelin gene, Sorghum kasirin-gene and rye secalin gene). Promoters suitable for preferential expression in plant root tissues include, for example, the promoter derived from corn nicotianamine synthase gene (US 20030131377) and rice RCC3 promoter (US 11/075,113). Suitable promoter for preferential expression in plant green tissues include the promoters from genes such as maize aldolase gene FDA (US 20040216189), aldolase and pyruvate orthophosphate dikinase (PPDK) (Taniguchi et. al., Plant Cell Physiol. 41(1):42-48, 2000).

"Inducible promoters" refer to those regulated promoters that can be turned on in one or more cell types by an external stimulus, for example, a chemical, light, hormone, stress, or a pathogen such as nematodes. Chemically inducible promoters are especially suitable if gene expression is wanted to occur in a time specific manner. Examples of such promoters are a salicylic acid inducible promoter (WO 95/19443), a tetracycline inducible promoter (Gatz et al., Plant J. 2:397-404, 1992), the light-inducible promoter from the small subunit of Ribulose-1,5-bis-phosphate carboxylase (ssRUBISCO), and an ethanol inducible promoter (WO 93/21334). Also, suitable promoters responding to biotic or abiotic stress conditions are those such as the pathogen inducible PRP1-gene promoter (Ward et al., Plant. Mol. Biol. 22:361-366, 1993), the heat inducible hsp80-promoter from tomato (US 5187267), cold inducible alpha-amylase promoter from potato (WO 96/12814), the drought-inducible promoter of maize (Busk et. al., Plant J. 11:1285-1295, 1997), the cold, drought, and high salt inducible promoter from potato (Kirch, Plant Mol. Biol. 33:897-909, 1997) or the RD29A promoter from Arabidopsis (Yamaguchi-Shinozalei et. al., Mol. Gen. Genet. 236:331-340, 1993), many cold inducible promoters such as cor15a promoter from Arabidopsis (Genbank Accession No U01377), but101 and blt4.8 from barley (Genbank Accession Nos AJ310994 and U63993), wcs120 from wheat (Genbank Accession No AF031235), mlip15 from corn (Genbank Accession No D26563), bn115 from Brassica (Genbank Accession No U01377), and the wound-inducible pinll-promoter (European Patent No. 375091).

Preferred promoters are root-specific, feeding site-specific, pathogen inducible or nematode inducible promoters.

Disclosed is a method of producing a transgenic plant comprising an alanine racemase encoding polynucleotide, wherein the method comprises the steps of introducing into the plant the expression vector comprising the alanine racemase encoding polynucleotide; and selecting transgenic plants for increased nematode resistance.

A variety of methods for introducing polynucleotides into the genome of plants and for the regeneration of plants from plant tissues or plant cells are known in, for example, Plant Molecular Biology and Biotechnology (CRC Press, Boca Raton, Florida), chapter 6/7, pp. 71-119 (1993); White FF (1993) Vectors for Gene Transfer in Higher Plants; Transgenic Plants, vol. 1, Engineering and Utilization, Ed.: Kung and Wu R, Academic Press, 15-38; Jenes B et al. (1993) Techniques for Gene Transfer; Transgenic Plants, vol. 1, Engineering and Utilization, Ed.: Kung and R. Wu, Academic Press, pp. 128-143; Potrykus (1991) Annu Rev Plant Physiol Plant Molec Biol 42:205-225; Halford NG, Shewry PR (2000) Br Med Bull 56(1):62-73.

Transformation methods may include direct and indirect methods of transformation. Suitable direct methods include polyethylene glycol induced DNA uptake, liposome-mediated transformation (US 4,536,475), biolistic methods using the gene gun (Fromm ME et al., Bio/Technology. 8(9):833-9, 1990; Gordon-Kamm et al., Plant Cell 2:603, 1990), electroporation, incubation of dry embryos in DNA-comprising solution, and microinjection. In the case of these direct transformation methods, the plasmid used need not meet any particular requirements. Simple plasmids, such as those of the pUC series, pBR322, M13mp series, pACYC184 and the like can be used. If intact plants are to be regenerated from the transformed cells, an additional selectable marker gene is preferably located on the plasmid. The direct transformation techniques are equally suitable for dicotyledonous and monocotyledonous plants.

Transformation can also be carried out by bacterial infection by means of Agrobacterium (for example EP 0 116 718), viral infection by means of viral vectors (EP 0 067 553; US 4,407,956; WO 95/34668; WO 93/03161) or by means of pollen (EP 0 270 356; WO 85/01856; US 4,684,611). Agrobacterium based transformation techniques (especially for dicotyledonous plants) are well known in the art. The Agrobacterium strain (e.g., Agrobacterium tumefaciens or Agrobacterium rhizogenes) comprises a plasmid (Ti or Ri plasmid) and a T-DNA element which is transferred to the plant following infection with Agrobacterium. The T-DNA (transferred DNA) is integrated into the genome of the plant cell. The T-DNA may be localized on the Ri- or Ti-plasmid or is separately comprised in a so-called binary vector. Methods for the Agrobacterium-mediated transformation are described, for example, in Horsch RB et al. (1985) Science 225:1229. The Agrobacterium-mediated transformation is best suited to dicotyledonous plants but has also been adopted to monocotyledonous plants. The transformation of plants by Agrobacteria is described in, for example, White FF, Vectors for Gene Transfer in Higher Plants, Transgenic Plants, Vol. 1, Engineering and Utilization, edited by S.D. Kung and R. Wu, Academic Press, 1993, pp. 15 - 38; Jenes B et al. Techniques for Gene Transfer, Transgenic Plants, Vol. 1, Engineering and Utilization, edited by S.D. Kung and R. Wu, Academic Press, 1993, pp. 128-143; Potrykus (1991) Annu Rev Plant Physiol Plant Molec Biol 42:205- 225.

Transformation may result in transient or stable transformation and expression. Although an alanine racemase encoding polynucleotide can be inserted into any plant or plant cell falling within these broad classes, it is particularly useful in crop plant cells.

Alanine racemase encoding polynucleotides can be directly transformed into the plastid genome. Plastid expression, in which genes are inserted by homologous recombination into the several thousand copies of the circular plastid genome present in each plant cell, takes advantage of the enormous copy number advantage over nuclear-expressed genes to permit high expression levels. In one embodiment, the nucleotides are inserted into a plastid targeting vector and transformed into the plastid genome of a desired plant host. Plants homoplasmic for plastid genomes containing the nucleotide sequences are obtained, and are preferentially capable of high expression of the nucleotides.

Plastid transformation technology is for example extensively described in U.S. Pat. NOs. 5,451,513, 5,545,817, 5,545,818, and 5,877,462 in WO 95/16783 and WO 97/32977, and in McBride et al. (1994) PNAS 91, 7301-7305, all incorporated herein by reference in their entirety. The basic technique for plastid transformation involves introducing regions of cloned plastid DNA flanking a selectable marker together with the nucleotide sequence into a suitable target tissue, e.g., using biolistic or protoplast transformation (e.g., calcium chloride or PEG mediated transformation). The 1 to 1.5 kb flanking regions, termed targeting sequences, facilitate homologous recombination with the plastid genome and thus allow the replacement or modification of specific regions of the plastome. Initially, point mutations in the chloroplast 16S rRNA and rps12 genes conferring resistance to spectinomycin and/or streptomycin are utilized as selectable markers for transformation (Svab et al., PNAS 87, 8526-8530, 1990; Staub et al., Plant Cell 4, 39-45, 1992). The presence of cloning sites between these markers allows creation of a plastid targeting vector for introduction of foreign genes (Staub et al., EMBO J. 12, 601-606, 1993). Substantial increases in transformation frequency are obtained by replacement of the recessive rRNA or r-protein antibiotic resistance genes with a dominant selectable marker, the bacterial aadA gene encoding the spectinomycin-detoxifying enzyme aminoglycoside-3'-adenyltransferase (Svab et al., PNAS 90, 913-917, 1993). Other selectable markers useful for plastid transformation are known in the art and encompassed within the scope of the invention.

The plant or transgenic plant may be any plant, such like, but not limited to trees, cut flowers, ornamentals, vegetables or crop plants. The plant may be from a genus selected from the group consisting of Medicago, Lycopersicon, Brassica, Cucumis, Solanum, Juglans, Gossypium, Malus, Vitis, Antirrhinum, Populus, Fragaria, Arabidopsis, Picea, Capsicum, Chenopodium, Dendranthema, Pharbitis, Pinus, Pisum, Oryza, Zea, Triticum, Triticale, Secale, Lolium, Hordeum, Glycine, Pseudotsuga, Kalanchoe, Beta, Helianthus, Nicotiana, Cucurbita, Rosa, Fragaria, Lotus, Medicago, Onobrychis, trifolium, Trigonella, Vigna, Citrus, Linum, Geranium, Manihot, Daucus, Raphanus, Sinapis, Atropa, Datura, Hyoscyamus, Nicotiana, Petunia, Digitalis, Majorana, Ciahorium, Lactuca, Bromus, Asparagus, Antirrhinum, Heterocallis, Nemesis, Pelargonium, Panieum, Pennisetum, Ranunculus, Senecio, Salpiglossis, Browaalia, Phaseolus, Avena, and Allium, or the plant may be selected from the group consisting of cereals including wheat, barley, sorghum, rye, triticale, maize, rice, sugarcane, and trees including apple, pear, quince, plum, cherry, peach, nectarine, apricot, papaya, mango, poplar, pine, sequoia, cedar, and oak. The term "plant" as used herein can be dicotyledonous crop plants, such as pea, alfalfa, soybean, carrot, celery, tomato, potato, cotton, tobacco, pepper, oilseed rape, beet, cabbage, cauliflower, broccoli, lettuce and Arabidopsis thaliana.,. In one embodiment the plant is a monocotyledonous plant or a dicotyledonous plant.

Preferably the plant is a crop plant. Crop plants are all plants, used in agriculture. Accordingly in one embodiment the plant is a monocotyledonous plant, preferably a plant of the family Poaceae, Musaceae, Liliaceae or Bromeliaceae, preferably of the family Poaceae. Accordingly, in yet another embodiment the plant is a Poaceae plant of the genus Zea, Triticum, Oryza, Hordeum, Secale, Avena, Saccharum, Sorghum, Pennisetum, Setaria, Panicum, Eleusine, Miscanthus, Brachypodium, Festuca or Lolium. When the plant is of the genus Zea, the preferred species is Z. mays. When the plant is of the genus Triticum, the preferred species is T. aestivum, T. speltae or T. durum. When the plant is of the genus Oryza, the preferred species is O. sativa. When the plant is of the genus Hordeum, the preferred species is H. vulgare. When the plant is of the genus Secale, the preferred species S. cereale. When the plant is of the genus Avena, the preferred species is A. sativa. When the plant is of the genus Saccarum, the preferred species is S. officinarum. When the plant is of the genus Sorghum, the preferred species is S. vulgare, S. bicolor or S. sudanense. When the plant is of the genus Pennisetum, the preferred species is P. glaucum. When the plant is of the genus Setaria, the preferred species is S. italica. When the plant is of the genus Panicum, the preferred species is P. miliaceum or P. virgatum. When the plant is of the genus Eleusine, the preferred species is E. coracana. When the plant is of the genus Miscanthus, the preferred species is M. sinensis. When the plant is a plant of the genus Festuca, the preferred species is F. arundinaria, F. rubra or F. pratensis. When the plant is of the genus Lolium, the preferred species is L. perenne or L. multiflorum. Alternatively, the plant may be Triticosecale.
Alternatively, in one embodiment the plant is a dicotyledonous plant, preferably a plant of the family Fabaceae, Solanaceae, Brassicaceae, Chenopodiaceae, Asteraceae, Malvaceae, Linacea, Euphorbiaceae, Convolvulaceae Rosaceae, Cucurbitaceae, Theaceae, Rubiaceae, Sterculiaceae or Citrus. In one embodiment the plant is a plant of the family Fabaceae, Solanaceae or Brassicaceae. Accordingly, in one embodiment the plant is of the family Fabaceae, preferably of the genus Glycine, Pisum, Arachis, Cicer, Vicia, Phaseolus, Lupinus, Medicago or Lens. Preferred species of the family Fabaceae are M. truncatula, M, sativa, G. max, P. sativum, A. hypogea, C. arietinum, V. faba, P. vulgaris, Lupinus albus, Lupinus luteus, Lupinus angustifolius or Lens culinaris. More preferred are the species G. max A. hypogea and M. sativa. Most preferred is the species G. max. When the plant is of the family Solanaceae, the preferred genus is Solanum, Lycopersicon, Nicotiana or Capsicum. Preferred species of the family Solanaceae are S. tuberosum, L. esculentum, N. tabaccum or C. chinense. More preferred is S. tuberosum. Accordingly, in one embodiment the plant is of the family Brassicaceae, preferably of the genus Brassica or Raphanus. Preferred species of the family Brassicaceae are the species B. napus, B. oleracea, B. juncea or B. rapa. More preferred is the species B. napus. When the plant is of the family Chenopodiaceae, the preferred genus is Beta and the preferred species is the B. vulgaris. When the plant is of the family Asteraceae, the preferred genus is Helianthus and the preferred species is H. annuus. When the plant is of the family Malvaceae, the preferred genus is Gossypium or Abelmoschus. When the genus is Gossypium, the preferred species is G. hirsutum or G. barbadense and the most preferred species is G. hirsutum. A preferred species of the genus Abelmoschus is the species A. esculentus. When the plant is of the family Linacea, the preferred genus is Linum and the preferred species is L. usitatissimum. When the plant is of the family Euphorbiaceae, the preferred genus is Manihot, Jatropa or Rhizinus and the preferred species are M. esculenta, J. curcas or R. comunis. When the plant is of the family Convolvulaceae, the preferred genus is Ipomea and the preferred species is I. batatas. When the plant is of the family Rosaceae, the preferred genus is Rosa, Malus, Pyrus, Prunus, Rubus, Ribes, Vaccinium or Fragaria and the preferred species is the hybrid Fragaria x ananassa. When the plant is of the family Cucurbitaceae, the preferred genus is Cucumis, Citrullus or Cucurbita and the preferred species is Cucumis sativus, Citrullus lanatus or Cucurbita pepo. When the plant is of the family Theaceae, the preferred genus is Camellia and the preferred species is C. sinensis. When the plant is of the family Rubiaceae, the preferred genus is Coffea and the preferred species is C. arabica or C. canephora. When the plant is of the family Sterculiaceae, the preferred genus is Theobroma and the preferred species is T. cacao. When the plant is of the genus Citrus, the preferred species is C. sinensis, C. limon, C. reticulata, C. maxima and hybrids of Citrus species, or the like. In a preferred embodiment of the invention, the plant is a soybean, a potato or a corn plant
The transgenic plants of the invention may be used in a method of controlling infestation of a crop by a plant parasitic nematode, which comprises the step of growing said crop from seeds comprising an expression cassette comprising a transcription regulatory element operably linked to a polynucleotide that encodes an alanine racemase, wherein the expression cassette is stably integrated into the genomes of the seeds and the plant has increased resistance to nematodes.
The invention also provides a method to confer nematode resistance to a plant, comprising the steps of a) transforming a plant cell with an expression cassette of the invention, b) regenerating a plant from that cell and c) selecting such plant for nematode resistance. More specifically, the method for increasing nematode resistance in a plant comprises the steps of introducing into the plant an expression vector comprising a transcription regulatory element operably linked to a polynucleotide of the invention, wherein expression of the polynucleotide confers increased nematode resistance to the plant, and wherein the alanine racemase encoding polynucleotide is selected from the group consisting of a polynucleotide having a sequence as defined in SEQ ID NO:5 or 7; a polynucleotide encoding a polypeptide having a sequence as defined in SEQ ID NO:6 or 8; a polynucleotide having at least 70% sequence identity to a polynucleotide having a sequence as defined in SEQ ID NO:5 or 7; a polynucleotide encoding a polypeptide having at least 70% sequence identity to a polypeptide having a sequence as defined in SEQ ID NO:6 or 8; a polynucleotide that hybridizes under stringent conditions to a polynucleotide having a sequence as defined in SEQ ID NO:5 or 7; a polynucleotide that hybridizes under stringent conditions to a polynucleotide encoding a polypeptide having a sequence as defined in SEQ ID NO:6 or 8; a polynucleotide encoding a polypeptide having a sequence as defined in any of SEQ ID NOs: 12 through 44, and a polynucleotide encoding a polypeptide having at least 90% sequence identity to any of the sequences as defined in any of SEQ ID NOs: 12 through 44.

The present invention may be used to reduce crop destruction by plant parasitic nematodes or to confer nematode resistance to a plant. The nematode may be any plant parasitic nematode, like nematodes of the families Longidoridae, Trichodoridae, Aphelenchoidida, Anguinidae, Belonolaimidae, Criconematidae, Heterodidae, Hoplolaimidae, Meloidogynidae, Paratylenchidae, Pratylenchidae, Tylenchulidae, Tylenchidae, or the like. Preferably, the parasitic nematodes belong to nematode families inducing giant or syncytial cells. Nematodes inducing giant or syncytial cells are found in the families Longidoridae, Trichodoridae, Heterodidae, Meloidogynidae, Pratylenchidae or Tylenchulidae. In particular in the families Heterodidae and Meloidogynidae.

Accordingly, parasitic nematodes targeted by the present invention belong to one or more genus selected from the group of Naccobus, Cactodera, Dolichodera, Globodera, Heterodera, Punctodera, Longidorus or Meloidogyne. In a preferred embodiment the parasitic nematodes belong to one or more genus selected from the group of Naccobus, Cactodera, Dolichodera, Globodera, Heterodera, Punctodera or Meloidogyne. In a more preferred embodiment the parasitic nematodes belong to one or more genus selected from the group of Globodera, Heterodera, or Meloidogyne. In an even more preferred embodiment the parasitic nematodes belong to one or both genus selected from the group of Globodera or Heterodera. In another embodiment the parasitic nematodes belong to the genus Meloidogyne.

When the parasitic nematodes are of the genus Globodera, the species are preferably from the group consisting of G. achilleae, G. artemisiae, G. hypolysi, G. mexicana, G. millefolii, G. mali, G. pallida, G. rostochiensis, G. tabacum, and G. virginiae. In another preferred embodiment the parasitic Globodera nematodes includes at least one of the species G. pallida, G. tabacum, or G. rostochiensis. When the parasitic nematodes are of the genus Heterodera, the species may be preferably from the group consisting of H. avenae, H. carotae, H. ciceri, H. cruciferae, H. delvii, H. elachista, H. filipjevi, H. gambiensis, H. glycines, H. goettingiana, H. graduni, H. humuli, H. hordecalis, H. latipons, H. major, H. medicaginis, H. oryzicola, H. pakistanensis, H. rosii, H. sacchari, H. schachtii, H. sorghi, H. trifolii, H. urticae, H. vigni and H. zeae. In another preferred embodiment the parasitic Heterodera nematodes include at least one of the species H. glycines, H. avenae, H. cajani, H. gottingiana, H. trifolii, H. zeae or H. schachtii. In a more preferred embodiment the parasitic nematodes includes at least one of the species H. glycines or H. schachtii. In a most preferred embodiment the parasitic nematode is the species H. glycines.

When the parasitic nematodes are of the genus Meloidogyne, the parasitic nematode may be selected from the group consisting of M. acronea, M. arabica, M. arenaria, M. artiellia, M. brevicauda, M. camelliae, M. chitwoodi, M. cofeicola, M. esigua, M. graminicola, M. hapla, M. incognita, M. indica, M. inornata, M. javanica, M. lini, M. mali, M. microcephala, M. microtyla, M. naasi, M. salasi and M. thamesi. In a preferred embodiment the parasitic nematodes includes at least one of the species M. javanica, M. incognita, M. hapla, M. arenaria or M. chitwoodi.

Accordingly the invention comprises a method of conferring nematode resistance to a plant, said method comprising the steps of: a) preparing an expression cassette comprising a polynucleotide of the invention operably linked to a promoter; b) transforming a recipient plant with said expression cassette; c) producing one or more transgenic offspring of said recipient plant; and d) selecting the offspring for nematode resistance. Preferably the promoter is a root-preferred or nematode inducible promoter or a promoter mediating expression in nematode feeding sites, e.g. syncytia or giant cells.

EXAMPLES

### Example 1: Cloning of alanine racemase encoding genes

The two forms of alanine racemase, Alr (SEQ ID NO:7) and DadX (Seq ID NO:5), were cloned from E.coli genomic DNA using PCR primers shown in Table 1.

**Table 1 Primers used to clone the ARLNCP coding genes**

| Primer name | Sequence | Purpose | SEQ ID NO: |
|---|---|---|---|
| Primer 1 -DadX | GCGGCGCGCCACCATGACCCGTCCGATACAGGC | DadX 5' primer | 1 |
| Primer 2 -DadX | GCCTCGAGTTACACCGTCACAACCGGGACGC | DadX 3' primer | 2 |
| Primer 1 - Alr | GCGGCGCGCCACCATGCAAGCGGCAACTGTTGTG | Alr 5' primer | 3 |
| Primer 2 - Alr | GCCTCGAGTTAATCCACGTATTTCATCGCGAC | Alr 3' primer | 4 |

### Example 2: Vector construction for transformation and generation of transgenic roots

PCR products generated in Example 1 were sequenced and cloned into a number of expression vectors containing syncytia preferred (nematode induced) promoters. The syncytia preferred promoters included soybean MTN3 SEQ ID NO:9 (p-47116125) (USSN 60/899,714), Arabidopsis peroxidase POX SEQ ID NO:10 (p-At5g05340) (USSN 60/876,416) and Arabidopsis TPP trehalose-6-phosphate phosphatase SEQ ID NO:11 (p-At1g35910) (USSN 60/874,375). The constitutive super promoter was also used. The selection marker for transformation was a mutated acetohydroxy acid synthase (AHAS) gene from Arabidopsis thaliana that conferred resistance to the herbicide ARSENAL (imazepyr, BASF Corporation, Mount Olive, NJ). The expression of mutated AHAS was driven by the Arabidopsis actin 2 promoter.

**Table 2. expression vector comprising SEQ ID NO:5 or 7**

| vector | Composition of the expression vector (promoter::ARLNCP encoding gene) |
|---|---|
| RSH118 | MTN3::DadX |
| RSH120 | POX::DadX |
| RSH122 | TPP::DadX |
| RSH117 | Super Promoter::DadX |
| RSH125 | MTN3::Alr |
| RSH127 | POX::Alr |
| RSH129 | TPP::Alr |
| RSH124 | Super Promoter::Alr |

### Example 3: Generation of transgenic soybean hairy-root and nematode bioassay

Vectors RSH118, RSH120, RSH122, RSH117, RSH125, RSH127, RSH129 and RSH124 were transformed into A. rhizogenes K599 strain by electroporation. The transformed strains of Agrobacterium were used to induce soybean hairy-root formation using known methods. Non-transgenic hairy roots from soybean cultivar Williams 82 (SCN susceptible) and Jack (SCN resistant) were also generated by using non-transformed A. rhizogenes, to serve as controls for nematode growth in the assay.

A bioassay to assess nematode resistance was performed on the transgenic hairy-root transformed with the vectors and on non-transgenic hairy roots from Williams 82 and Jack as controls. Hairy root cultures of each line that occupied at least half of the well were inoculated with surface-decontaminated race 3 of soybean cyst nematode (SCN) second stage juveniles (J2). The plates were then sealed and put back into the incubator at 25°C in darkness. Several independent hairy root lines were generated from each binary vector transformation and the lines used for bioassay. Four weeks after nematode inoculation, the cyst number in each well was counted.

Bioassay results for constructs RSH118, RSH120, RSH122, and RSH125 show a statistically significant reduction (p-value <0.05) in cyst count over multiple transgenic lines and a general trend of reduced cyst count in the majority of transgenic lines tested.

Those skilled in the art will recognize, or will be able to ascertain using no more than routine experimentation, many equivalents to the specific embodiments of the invention described herein.

## Claims

1. A transgenic plant comprising an expression vector comprising an isolated alanine racemase encoding polynucleotide, wherein the transgenic plant demonstrates increased resistance to nematodes as compared to a wild type plant.

2. The transgenic plant of claim 1, wherein the isolated polynucleotide is selected from the group consisting of:
a) a polynucleotide having a sequence as defined in SEQ ID NO:5 or 7;
b) a polynucleotide encoding a polypeptide having a sequence as defined in SEQ ID NO:6 or 8;
c) a polynucleotide having at least 70% sequence identity to a polynucleotide having a sequence as defined in SEQ ID NO:5 or 7;
d) a polynucleotide encoding a polypeptide having at least 70% sequence identity to a polypeptide having a sequence as defined in SEQ ID NO:6 or 8;
e) a polynucleotide that hybridizes under stringent conditions to a polynucleotide having a sequence as defined in SEQ ID NO:5 or 7;
f) a polynucleotide that hybridizes under stringent conditions to a polynucleotide encoding a polypeptide having a sequence as defined in SEQ ID NO:6 or 8;
g) a polynucleotide encoding a polypeptide having a sequence as defined in any of SEQ ID NOs: 12 through 44, and
h) a polynucleotide encoding a polypeptide having at least 90% sequence identity to a sequence as defined in any of SEQ ID NO: 12 through 44.

3. The plant of claim 1, further defined as a monocot or a dicot.

4. The plant of claim 1, wherein the plant is selected from the group consisting of maize, wheat, rice, barley, oat, rye, sorghum, banana, ryegrass, pea, alfalfa, soybean, carrot, celery, tomato, potato, cotton, tobacco, pepper, oilseed rape, beet, cabbage, cauliflower, broccoli, lettuce and *Arabidopsis thaliana.*

5. A seed which is true breeding for a transgene comprising an alanine racemase encoding polynucleotide, wherein expression of the polynucleotide confers increased nematode resistance to the plant produced from the seed.

6. The seed of claim 5, wherein the polynucleotide is selected from the group consisting of:
a) a polynucleotide having a sequence as defined in SEQ ID NO:5 or 7;
b) a polynucleotide encoding a polypeptide having a sequence as defined in SEQ ID NO:6 or 8;
c) a polynucleotide having at least 70% sequence identity to a polynucleotide having a sequence as defined in SEQ ID NO:5 or 7;
d) a polynucleotide encoding a polypeptide having at least 70% sequence identity to a polypeptide having a sequence as defined in SEQ ID NO:6 or 8;
e) a polynucleotide that hybridizes under stringent conditions to a polynucleotide having a sequence as defined in SEQ ID NO:5 or 7;
f) a polynucleotide that hybridizes under stringent conditions to a polynucleotide encoding a polypeptide having a sequence as defined in SEQ ID NO:6 or 8;
g) a polynucleotide encoding a polypeptide having a sequence as defined in any of SEQ ID NOs: 12 through 44, and
h) a polynucleotide encoding a polypeptide having at least 90% sequence identity to a sequence as defined in any of SEQ ID NO: 12 through 44.

7. Use of an expression vector comprising a transcription regulatory element operably linked to an alanine racemase encoding polynucleotide for increasing nematode resistance of a plant.

8. The use of claim 7, wherein the polynucleotide is selected from the group consisting of:
a) a polynucleotide having a sequence as defined in SEQ ID NO:5 or 7;
b) a polynucleotide encoding a polypeptide having a sequence as defined in SEQ ID NO:6 or 8;
c) a polynucleotide having at least 70% sequence identity to a polynucleotide having a sequence as defined in SEQ ID NO:5 or 7;
d) a polynucleotide encoding a polypeptide having a sequence as defined in SEQ ID NO:6 or 8;
e) a polynucleotide that hybridizes under stringent conditions to a polynucleotide having a sequence as defined in SEQ ID NO:5 or 7; and
f) a polynucleotide that hybridizes under stringent conditions to a polynucleotide encoding a polypeptide having a sequence as defined in SEQ ID NO:6 or 8;
g) a polynucleotide encoding a polypeptide having a sequence as defined in any of SEQ ID NOs: 12 through 44, and
h) a polynucleotide encoding a polypeptide having at least 90% sequence identity to a sequence as defined in any of SEQ ID NOs: 12 through 44.

9. The use of claim 8, wherein the transcription regulatory element is a promoter regulating root-specific or syncytia-specific expression of the polynucleotide.

10. A method for increasing nematode resistance of a plant, wherein the method comprises the steps of:
a) introducing into the plant the expression vector comprising an alanine racemase encoding polynucleotide; and
b) selecting transgenic plants with increased nematode resistance.

11. The method of claim 10, wherein the plant is a monocot.

12. The method of claim 11, wherein the plant is selected from the group consisting of maize, wheat, rice, barley, oat, rye, sorghum, banana, and ryegrass.

13. The method of claim 10, wherein the plant is a dicot.

14. The method of claim 13, wherein the plant is selected from the group consisting of pea, alfalfa, soybean, carrot, celery, tomato, potato, cotton, tobacco, pepper, oilseed rape, beet, cabbage, cauliflower, broccoli, lettuce and *Arabidopsis thaliana.*

15. The method of claim 10, wherein the polynucleotide has a sequence as defined in SEQ ID NO:5 or 7.

16. The method of claim 10, wherein the polynucleotide encodes a polypeptide having a sequence as defined in SEQ ID NO:6 or 8.

## Patentansprüche

1. Transgene Pflanze, die einen Expressionsvektor umfasst, der ein isoliertes für Alaninracemase codierendes Polynukleotid umfasst, wobei die transgene Pflanze im Vergleich zu einer Wildtyppflanze eine erhöhte Nematodenresistenz aufweist.

2. Transgene Pflanze nach Anspruch 1, wobei das isolierte Polynukleotid aus der folgenden Gruppe ausgewählt ist:
a) Polynukleotid mit einer Sequenz gemäß SEQ ID NO: 5 oder 7;
b) Polynukleotid, das für ein Polypeptid mit einer Sequenz gemäß SEQ ID NO: 6 oder 8 codiert;
c) Polynukleotid mit mindestens 70% Sequenzidentität zu einem Polynukleotid mit einer Sequenz gemäß SEQ ID NO: 5 oder 7;
d) Polynukleotid, das für ein Polypeptid mit mindestens 70% Sequenzidentität zu einem Polypeptid mit einer Sequenz gemäß SEQ ID NO: 6 oder 8 codiert;
e) Polynukleotid, das unter stringenten Bedingungen mit einem Polynukleotid mit einer Sequenz gemäß SEQ ID NO: 5 oder 7 hybridisiert;
f) Polynukleotid, das unter stringenten Bedingungen mit einem Polynukleotid hybridisiert, das für ein Polypeptid mit einer Sequenz gemäß SEQ ID NO: 6 oder 8 codiert;
g) Polynukleotid, das für ein Polypeptid mit einer Sequenz gemäß einem der SEQ ID NO: 12 bis 44 codiert, und
h) Polynukleotid, das für ein Polypeptid mit mindestens 90% Sequenzidentität zu einer Sequenz nach einem der SEQ ID NO: 12 bis 44 codiert.

3. Pflanze nach Anspruch 1, die weiterhin als monokotyle oder dikotyle Pflanze definiert ist.

4. Pflanze nach Anspruch 1, wobei die Pflanze aus der Gruppe Mais, Weizen, Reis, Gerste, Hafer, Roggen, Sorghum, Banane, Raigras, Erbse, Luzerne, Sojabohne, Karotte, Sellerie, Tomate, Kartoffel, Baumwolle, Tabak, Pfeffer, Raps, Rübe, Kohl, Blumenkohl, Brokkoli, Salat und *Arabidopsis thaliana* stammt.

5. Samen, der für ein Transgen reinerbig ist, das ein für Alaninracemase codierendes Polynukleotid umfasst, wobei die Expression des Polynukleotids der aus dem Samen erzeugten Pflanze eine erhöhte Nematodenresistenz verleiht.

6. Samen nach Anspruch 5, wobei das Polynukleotid aus der folgenden Gruppe stammt:
a) Polynukleotid mit einer Sequenz gemäß SEQ ID NO: 5 oder 7;
b) Polynukleotid, das für ein Polypeptid mit einer Sequenz gemäß SEQ ID NO: 6 oder 8 codiert;
c) Polynukleotid mit mindestens 70% Sequenzidentität zu einem Polynukleotid mit einer Sequenz gemäß SEQ ID NO: 5 oder 7;
d) Polynukleotid, das für ein Polypeptid mit mindestens 70% Sequenzidentität zu einem Polypeptid mit einer Sequenz gemäß SEQ ID NO: oder 8 codiert;
e) Polynukleotid, das unter stringenten Bedingungen mit einem Polynukleotid mit einer Sequenz gemäß SEQ ID NO: 5 oder 7 hybridisiert;
f) Polynukleotid, das unter stringenten Bedingungen mit einem Polynukleotid hybridisiert, das für ein Polypeptid mit einer Sequenz gemäß SEQ ID NO: 6 oder 8 codiert;
g) Polynukleotid, das für ein Polypeptid mit einer Sequenz gemäß einem der SEQ ID NO: 12 bis 44 codiert, und
h) Polynukleotid, das für ein Polypeptid mit mindestens 90% Sequenzidentität zu einer Sequenz nach einem der SEQ ID NO: 12 bis 44 codiert.

7. Verwendung eines Expressionsvektors, der ein Transkriptionsregulationselement, das operativ mit einem für Alaninracemase codierenden Polynukleotid verknüpft ist, umfasst, zum Erhöhen der Nematodenresistenz einer Pflanze.

8. Verwendung nach Anspruch 7, wobei das Polynukleotid aus der folgenden Gruppe stammt:
a) Polynukleotid mit einer Sequenz gemäß SEQ ID NO: 5 oder 7;
b) Polynukleotid, das für ein Polypeptid mit einer Sequenz gemäß SEQ ID NO: 6 oder 8 codiert;
c) Polynukleotid mit mindestens 70% Sequenzidentität zu einem Polynukleotid mit einer Sequenz gemäß SEQ ID NO: 5 oder 7;
d) Polynukleotid, das für ein Polypeptid mit mindestens 70% Sequenzidentität zu einem Polypeptid mit einer Sequenz gemäß SEQ ID NO: 6 oder 8 codiert;
e) Polynukleotid, das unter stringenten Bedingungen mit einem Polynukleotid mit einer Sequenz gemäß SEQ ID NO: 5 oder 7 hybridisiert; und
f) Polynukleotid, das unter stringenten Bedingungen mit einem Polynukleotid hybridisiert, das für ein Polypeptid mit einer Sequenz gemäß SEQ ID NO: 6 oder 8 codiert;
g) Polynukleotid, das für ein Polypeptid mit einer Sequenz gemäß einem der SEQ ID NO: 12 bis 44 codiert, und
h) Polynukleotid, das für ein Polypeptid mit mindestens 90% Sequenzidentität zu einer Sequenz nach einem der SEQ ID NO: 12 bis 44 codiert.

9. Verwendung nach Anspruch 8, wobei es sich bei dem Transkriptionsregulationselement um einen Promoter, der eine wurzelspezifische oder syncytienspezifische Expression des Polynukleotids reguliert, handelt.

10. Verfahren zum Erhöhen der Nematodenresistenz einer Pflanze, wobei das Verfahren die folgenden Schritte umfasst:
a) Einführen des Expressionsvektors, der ein für Alaninracemase codierendes Polynukleotid umfasst, in die Pflanze; und
b) Selektieren von transgenen Pflanzen mit erhöhter Nematodenresistenz.

11. Verfahren nach Anspruch 10, wobei es sich bei der Pflanze um eine monokotyle Pflanze handelt.

12. Verfahren nach Anspruch 11, wobei die Pflanze aus der Gruppe Mais, Weizen, Reis, Gerste, Hafer, Roggen, Sorghum, Banane und Raigras stammt.

13. Verfahren nach Anspruch 10, wobei es sich bei der Pflanze um eine dikotyle Pflanze handelt.

14. Verfahren nach Anspruch 13, wobei die Pflanze aus der Gruppe Erbse, Luzerne, Sojabohne, Karotte, Sellerie, Tomate, Kartoffel, Baumwolle, Tabak, Pfeffer, Raps, Rübe, Kohl, Blumenkohl, Brokkoli, Salat und *Arabidopsis thaliana* stammt.

15. Verfahren nach Anspruch 10, wobei das Polynukleotid eine Sequenz gemäß SEQ ID NO: 5 oder 7 aufweist.

16. Verfahren nach Anspruch 10, wobei das Polynukleotid für ein Polypeptid mit einer Sequenz gemäß SEQ ID NO: 6 oder 8 codiert.

## Revendications

1. Plante transgénique comprenant un nucléotide isolé codant pour l'alanine racémase, la plante transgénique présentant une résistance aux nématodes, accrue par comparaison avec celle d'une plante de type sauvage.

2. Plante transgénique de la revendication 1, dans laquelle le polynucléotide isolé est choisi dans le groupe consistant en :
a) un polynucléotide ayant une séquence telle que définie dans SEQ ID N° 5 ou 7 ;
b) un polynucléotide codant pour un polypeptide ayant une séquence telle que définie dans SEQ ID N° 6 ou 8 ;
c) un polynucléotide ayant une identité de séquence d'au moins 70 % avec un polynucléotide ayant une séquence telle que définie dans SEQ ID N° 5 ou 7 ;
d) un polynucléotide codant pour un polypeptide ayant une identité de séquence d'au moins 70 % avec un polypeptide ayant une séquence telle que définie dans SEQ ID N° 6 ou 8 ;
e) un polynucléotide qui s'hybride dans des conditions stringentes à un polynucléotide ayant une séquence telle que définie dans SEQ ID N° 5 ou 7 ;
f) un polynucléotide qui s'hybride dans des conditions stringentes à un polynucléotide codant pour un polypeptide ayant une séquence telle que définie dans SEQ ID N° 6 ou 8 ;
g) un polynucléotide codant pour un polypeptide ayant une séquence telle que définie dans l'une quelconque des séquences SEQ ID N° 12 à 44, et
h) un polynucléotide codant pour un polypeptide ayant une identité de séquence d'au moins 90 % avec une séquence telle que définie dans l'une quelconque des séquences SEQ ID N° 12 à 44.

3. Plante de la revendication 1, définie en outre comme étant une plante monocotylédone ou dicotylédone.

4. Plante de la revendication 1, la plante étant choisie dans le groupe consistant en le maïs, le blé, le riz, l'orge, l'avoine, le seigle, le sorgho, la banane, le raygrass, le pois, la luzerne, le soja, la carotte, le céleri, la tomate, la pomme de terre, le coton, le tabac, le poivre, le colza oléagineux, la betterave, le chou, le chou-fleur, le brocoli, la laitue et *Arabidopsis thaliana.*

5. Graine qui est une lignée pure pour un transgène comprenant un polynucléotide codant pour l'alanine racémase, dans laquelle l'expression du polynucléotide confère à la plante produite à partir de la graine une résistance accrue aux nématodes.

6. Graine de la revendication 5, dans laquelle le polynucléotide est choisi dans le groupe consistant en :
a) un polynucléotide ayant une séquence telle que définie dans SEQ ID N° 5 ou 7 ;
b) un polynucléotide codant pour un polypeptide ayant une séquence telle que définie dans SEQ ID N° 6 ou 8 ;
c) un polynucléotide ayant une identité de séquence d'au moins 70 % avec un polynucléotide ayant une séquence telle que définie dans SEQ ID N° 5 ou 7 ;
d) un polynucléotide codant pour un polypeptide ayant une identité de séquence d'au moins 70 % avec un polypeptide ayant une séquence telle que définie dans SEQ ID N° 6 ou 8 ;
e) un polynucléotide qui s'hybride dans des conditions stringentes à un polynucléotide ayant une séquence telle que définie dans SEQ ID N° 5 ou 7 ;
f) un polynucléotide qui s'hybride dans des conditions stringentes à un polynucléotide codant pour un polypeptide ayant une séquence telle que définie dans SEQ ID N° 6 ou 8 ;
g) un polynucléotide codant pour un polypeptide ayant une séquence telle que définie dans l'une quelconque des séquences SEQ ID N° 12 à 44, et
h) un polynucléotide codant pour un polypeptide ayant une identité de séquence d'au moins 90 % avec une séquence telle que définie dans l'une quelconque des séquences SEQ ID N° 12 à 44.

7. Utilisation d'un vecteur d'expression comprenant un élément régulateur de transcription lié d'une manière opérationnelle à un polynucléotide codant pour l'alanine racémase, pour accroître la résistance d'une plante aux nématodes.

8. Utilisation de la revendication 7, pour laquelle le polynucléotide est choisi dans le groupe consistant en :
a) un polynucléotide ayant une séquence telle que définie dans SEQ ID N° 5 ou 7 ;
b) un polynucléotide codant pour un polypeptide ayant une séquence telle que définie dans SEQ ID N° 6 ou 8 ;
c) un polynucléotide ayant une identité de séquence d'au moins 70 % avec un polynucléotide ayant une séquence telle que définie dans SEQ ID N° 5 ou 7 ;
d) un polynucléotide codant pour un polypeptide ayant une séquence telle que définie dans SEQ ID N° 6 ou 8 ;
e) un polynucléotide qui s'hybride dans des conditions stringentes à un polynucléotide ayant une séquence telle que définie dans SEQ ID N° 5 ou 7 ; et
f) un polynucléotide qui s'hybride dans des conditions stringentes à un polynucléotide codant pour un polypeptide ayant une séquence telle que définie dans SEQ ID N° 6 ou 8 ;
g) un polynucléotide codant pour un polypeptide ayant une séquence telle que définie dans l'une quelconque des séquences SEQ ID N° 12 à 44, et
h) un polynucléotide codant pour un polypeptide ayant une identité de séquence d'au moins 90 % avec une séquence telle que définie dans l'une quelconque des séquences SEQ ID N° 12 à 44.

9. Utilisation de la revendication 8, pour laquelle l'élément régulateur de transcription est un promoteur régulant l'expression spécifique de racine ou spécifique de syncytium du polynucléotide.

10. Procédé pour accroître la résistance d'une plante aux nématodes, le procédé comprenant les étapes de :
a) introduction, dans la plante, du vecteur d'expression comprenant un polynucléotide codant pour l'alanine racémase ; et
b) sélection des plantes transgéniques ayant une résistance accrue aux nématodes.

11. Procédé de la revendication 10, dans lequel la plante est une monocotylédone.

12. Procédé de la revendication 11, dans lequel la plante est choisie dans le groupe consistant en le maïs, le blé, le riz, l'orge, l'avoine, le seigle, le sorgho, la banane et le raygrass.

13. Procédé de la revendication 10, dans lequel la plante est une dicotylédone.

14. Procédé de la revendication 13, dans lequel la plante est choisie dans le groupe consistant en le pois, la luzerne, le soja, la carotte, le céleri, la tomate, la pomme de terre, le coton, le tabac, le poivre, le colza oléagineux, la betterave, le chou, le chou-fleur, le brocoli, la laitue et *Arabidopsis thaliana.*

15. Procédé de la revendication 10, dans lequel le polynucléotide a une séquence telle que définie dans SEQ ID N° 5 ou 7.

16. Procédé de la revendication 10, dans lequel le polynucléotide code pour un polypeptide ayant une séquence telle que définie dans SEQ ID N° 6 ou 8.
